# EUROPEAN PATENT SPECIFICATION

(11) **EP 3 756 532 B1**
(45) Date of publication and mention of the grant of the patent: **17.04.2024**
(21) Application number: 19758073.1
(22) Date of filing: 07.02.2019
(51) Int. Cl.: A61B 1/00, A61B 1/06, A61B 5/00, H04N 23/50

(54) **ENDOSCOPE SYSTEM**
ENDOSKOPSYSTEM
SYSTÈME D'ENDOSCOPE

(30) Priority: 20.02.2018 JP 2018027748
(43) Date of publication of application: 30.12.2020
(73) Proprietor: FUJIFILM Corporation, Tokyo 106-8620 (JP)
(72) Inventor: IWANE, Kosuke, Ashigarakami-gun, Kanagawa 258-8538 (JP)
(74) Representative: Hughes, Andrea Michelle
(86) International application number: PCT/JP2019/004486
(87) International publication number: WO 2019/163540

(56) References cited:
- EP-A1- 3 711 652
- WO-A1-2016/006427
- WO-A1-2017/057574
- JP-A- 2016 067 780
- JP-A- 2016 131 837

## Description

### BACKGROUND OF THE INVENTION

### 1. Field of the Invention

The present invention relates to an endoscope system that switches and displays a plurality of kinds of images.

### 2. Description of the Related Art

In recent years, an endoscope system comprising a light source device, an endoscope, and a processor device has been widely used in a medical field. In the endoscope system, an object to be observed is irradiated with illumination light from an endoscope, and the image of the object to be observed is displayed on a monitor on the basis of RGB image signals that are obtained in a case where the image of the object to be observed, which is being illuminated with the illumination light, is picked up by an image pickup element of the endoscope.

In recent years, a plurality of observation images having different display contents have been simultaneously displayed or switched and displayed on a monitor according to the purpose of diagnosis. A plurality of images of which the thicknesses and depths of blood vessels are different from each other are switched and displayed in, for example, JP2017-060682A. Further, in JP2016-067780A, an image corresponding to violet light V and an image corresponding to blue light B are acquired by illumination using the violet light V and the blue light B that are being switched, the image corresponding to the violet light V is assigned to brightness signals in a case where extremely superficial blood vessels are to be emphasized, and the image corresponding to the blue light B is assigned to brightness signals in a case where superficial blood vessels are to be emphasized. JP2012-147928A discloses the preamble of claim 1.

### SUMMARY OF THE INVENTION

In recent years, a diagnosis focusing on information other than a background mucous membrane, for example, blood vessels having different depths, glandular structures having different depths or heights, or the like has been made in an endoscopic field. A plurality of kinds of information other than the background mucous membrane need to be displayed in such a diagnosis so that a user can grasp the information. A method of switching and displaying a plurality of images as disclosed in JP2017-060682A is considered as a method of displaying each of the plurality of kinds of information. In a case where a plurality of images are switched and displayed as described above, a background mucous membrane needs to be displayed so as to have the same color tone in every image so that a difference in information other than the background mucous membrane is recognized through the respective images of the background mucous membrane. However, JP2017-060682A does not disclose and imply that the background mucous membrane is made to have the same color tone in every image. Accordingly, even though the images are switched and displayed, there is a case where a user does not recognize a difference in the depths or thicknesses of blood vessels. WO 2017/057574 A1 discloses an image processing apparatus, endoscope system and image processing method.

An object of the invention is to provide an endoscope system that can display a plurality of observation images to allow a user to recognize a difference in information other than a background mucous membrane in a case where the plurality of observation images are switched and displayed.

According to a first aspect of the present invention, there is provided an endoscope system as claimed in claim 1.

The endoscope system according to the first aspect of the invention further includes a light source unit that emits first illumination light having the first spectral information and second illumination light having the second spectral information and a light source control unit that causes each of the first illumination light and the second illumination light to be emitted for a light emission period of at least two or more frames and automatically switches the first illumination light and the second illumination light. The first observation image for display is obtained in a case where an image of the object to be observed illuminated with the first illumination light is picked up and the second observation image for display is obtained in a case where an image of the object to be observed illuminated with the second illumination light is picked up.

It is preferable that the display control unit causes the display unit to display a third observation image for display different from the first observation image for display and the second observation image for display at a timing when the first observation image for display and the second observation image for display are switched. It is preferable that the light source control unit causes third illumination light different from the first illumination light and the second illumination light to be emitted at a timing when the first observation image for display and the second observation image for display are switched.

It is preferable that the first illumination light includes violet light, green light, and red light and the second illumination light includes green light and red light. It is preferable that the first illumination light includes violet light, green light, and red light and the second illumination light includes blue light, green light, and red light.

It is preferable that the first observation image for display includes first specific information, which is included in a first depth of biological tissue, in a region thereof other than the background mucous membrane, the second observation image for display includes second specific information, which is included in a second depth of the biological tissue, in a region thereof other than the background mucous membrane, and the second depth is deeper than the first depth. It is preferable that the first illumination light includes first depth-reaching light reaching the first depth, and the second illumination light includes second depth-reaching light reaching the second depth.

It is preferable that the endoscope system according to the aspect of the invention further comprises an observation-image-for-display processing unit that generates an observation image for display on the basis of the observation image. It is preferable that the observation-image-for-display processing unit assigns a first color signal of the observation image to lightness information to generate the first observation image for display as the observation image for display, and assigns a second color signal of the observation image to lightness information to generate the second observation image for display as the observation image for display. It is preferable that the observation image is obtained in a case where an image of the object to be observed illuminated with special light including violet light, green light, and red light is picked up.

It is preferable that the display control unit causes the display unit to display a third observation image for display different from the first observation image for display and the second observation image for display at a timing when the first observation image for display and the second observation image for display are switched. It is preferable that the observation-image-for-display processing unit assigns a composite signal, which is obtained by combination of the first color signal and the second color signal of the observation image, to lightness information to generate the third observation image for display at a timing when the first observation image for display and the second observation image for display are switched.

It is preferable that the first observation image for display includes first specific information, which is included in a first depth of biological tissue, in a region thereof other than the background mucous membrane, the second observation image for display includes second specific information, which is included in a second depth of the biological tissue, in a region thereof other than the background mucous membrane, and the second depth is deeper than the first depth. It is preferable that the first spectral information includes first depth-spectral information corresponding to the first depth and the second spectral information includes second depth-spectral information corresponding to the second depth.

It is preferable that a parameter for the first observation image for display used for the generation of the first observation image for display and a parameter for the second observation image for display used for the generation of the second observation image for display are switched according to switching of the first observation image for display and the second observation image for display.

It is preferable that the first observation image for display includes first specific information, which is included in a first depth of biological tissue, in a region thereof other than the background mucous membrane and the second observation image for display includes second specific information, which is included in a second depth of the biological tissue deeper than the first depth, in a region thereof other than the background mucous membrane, and first reduction processing for reducing the second specific information from the first observation image for display or second reduction processing for reducing the first specific information from the second observation image for display is performed.

It is preferable that the display control unit includes a display period-setting unit that sets a display period of the first observation image for display and a display period of the second observation image for display. It is preferable that the endoscope system according to the aspect of the invention further includes a static image-acquisition instruction unit that issues a static image-acquisition instruction, and a static image-storage control unit that performs control to store a set of observation images for display, which includes the first observation image for display and the second observation image for display, in a static image storage unit in a case where the static image-acquisition instruction is issued.

It is preferable that the first specific information is first depth-blood vessels positioned at the first depth, and the second specific information is second depth-blood vessels positioned at the second depth. It is preferable that the first depth-blood vessels extracted by blood vessel-extraction processing are displayed in the first observation image for display, and the second depth-blood vessels extracted by the blood vessel-extraction processing are displayed in the second observation image for display. It is preferable that the first specific information is first depth-glandular structures positioned at the first depth, and the second specific information is second depth-glandular structures positioned at the second depth.

According to the invention, it is possible to display a plurality of observation images to allow a user to recognize a difference in information other than a background mucous membrane in a case where the plurality of observation images are switched and displayed.

### BRIEF DESCRIPTION OF THE DRAWINGS

Fig. 1 is a diagram showing the appearance of an endoscope system according to a first embodiment.
Fig. 2 is a block diagram showing the functions of the endoscope system according to the first embodiment.
Fig. 3 is a graph showing the emission spectra of violet light V, blue light B, green light G, and red light R.
Fig. 4 is a graph showing the emission spectrum of first illumination light that includes violet light V, green light G, and red light R.
Fig. 5 is a graph showing the emission spectrum of second illumination light that includes green light G and red light R.
Fig. 6 is a diagram showing the light emission period of the first illumination light and the light emission period of the second illumination light.
Fig. 7 is a diagram showing a light emission period-setting menu.
Fig. 8 is a graph showing the emission spectrum of third illumination light that includes violet light V, green light G, and red light R.
Fig. 9 is an image diagram showing a first observation image for display of the first embodiment.
Fig. 10 is a diagram showing a relationship between the first illumination light and a subject image (including blood vessels) displayed as the first observation image for display.
Fig. 11 is a diagram showing a relationship between the first illumination light and subject images (including blood vessels) that are included in reflected components of the first illumination light.
Fig. 12 is an image diagram showing a second observation image for display of the first embodiment.
Fig. 13 is a diagram showing a relationship between the second illumination light that includes green light G and red light R and a subject image displayed as the second observation image for display.
Fig. 14 is a diagram showing a relationship between the second illumination light that includes green light G and red light R and subject images that are included in reflected components of the second illumination light.
Fig. 15 is a diagram showing the switching display of the first and second observation images for display that are color images.
Fig. 16 is a diagram showing a third observation image for display that is displayed at the time of the switching of the first and second observation images for display.
Fig. 17 is a diagram showing the switching display of the first and second observation images for display that are monochrome images.
Fig. 18 is a diagram showing a display period-setting menu.
Fig. 19 is a graph showing the emission spectrum of second illumination light that includes blue light B, green light G, and red light R.
Fig. 20 is a diagram showing a relationship that the second illumination light that includes blue light B, green light G, and red light R and subject images that are included in reflected components of the second illumination light.
Fig. 21A is a diagram showing a relationship between the first illumination light and subject images (including glandular structures) that are included in reflected components of the first illumination light.
Fig. 21B is an image diagram of the first observation image for display that displays first depth-glandular structures and second depth-glandular structures.
Fig. 22A is a diagram showing a relationship between the second illumination light that includes blue light B, green light G, and the red light R and subject images (including glandular structures) that are included in reflected components of the second illumination light.
Fig. 22B is an image diagram of the second observation image for display that displays the first depth-glandular structures and the second depth-glandular structures.
Fig. 23 is a block diagram showing the functions of an endoscope system according to another aspect of the first embodiment.
Fig. 24 is a block diagram showing the functions of an endoscope system according to a second embodiment.
Fig. 25 is a graph showing the emission spectrum of special light that includes violet light V, green light G, and red light R.
Fig. 26 is a diagram showing first-observation-image-for-display generation processing.
Fig. 27 is an image diagram showing a first observation image for display of the second embodiment.
Fig. 28 is a diagram showing second-observation-image-for-display generation processing.
Fig. 29 is an image diagram showing a second observation image for display of the second embodiment.
Fig. 30 is a diagram showing third-observation-image-for-display generation processing.
Fig. 31 is an image diagram showing a third observation image for display of the second embodiment.
Fig. 32 is a diagram showing the spectrum of special light, the reflectivity of an object to be observed, and a relationship between the transmittance of the respective pixels of an image pickup sensor and a Bs-image signal, a Gs-image signal, and an Rs-image signal.
Fig. 33 is a diagram showing a relationship between components of the reflected light of special light and a Bs-image signal and a Gs-image signal.
Fig. 34 is a diagram showing that any one of the first observation image for display or the second observation image for display is generated from a special observation image of one frame and the generated observation image for display is displayed.
Fig. 35 is a diagram showing that both the first and second observation images for display are generated from a special observation image of one frame and any one of the generated observation images for display is displayed.
Fig. 36 is a diagram showing first-observation-image-for-display generation processing in a case where first arithmetic processing of a second embodiment is performed.
Fig. 37 is a diagram showing first arithmetic processing of the second embodiment.
Fig. 38 is a diagram showing first arithmetic processing of the first embodiment.
Fig. 39 is a diagram showing blood vessel-extraction processing of the first embodiment.
Fig. 40 is a blood vessel-extraction processing of the second embodiment.

### DESCRIPTION OF THE PREFERRED EMBODIMENTS

### [First embodiment]

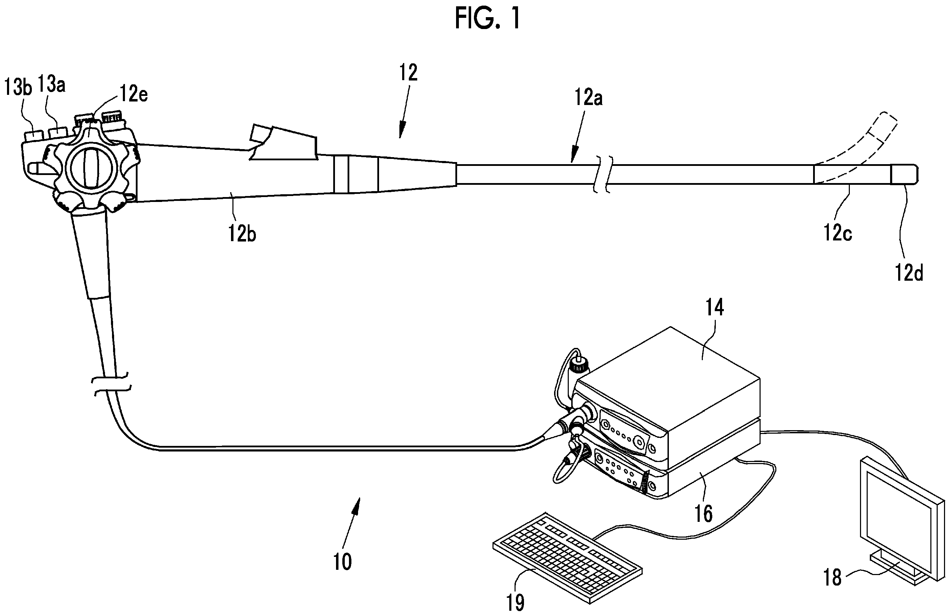

As shown in Fig. 1, an endoscope system 10 according to a first embodiment includes an endoscope 12, a light source device 14, a processor device 16, a monitor 18, and a user interface 19. The endoscope 12 is optically connected to the light source device 14, and is electrically connected to the processor device 16. The endoscope 12 includes an insertion part 12a that is to be inserted into an object to be examined, an operation part 12b that is provided at the proximal end portion of the insertion part 12a, and a bendable part 12c and a distal end part 12d that are provided on the distal end side of the insertion part 12a. In a case where angle knobs 12e of the operation part 12b are operated, the bendable part 12c is operated to be bent. As the bendable part 12c is operated to be bent, the distal end part 12d faces in a desired direction. The user interface 19 includes a mouse and the like in addition to a keyboard shown in Fig. 1.

Further, the operation part 12b is provided with a mode changeover SW 13a and a static image-acquisition instruction unit 13b in addition to the angle knobs 12e. The mode changeover SW 13a is used for an operation for switching a normal observation mode, a first special observation mode, a second special observation mode, and a multi-observation mode. The normal observation mode is a mode where a normal image is displayed on the monitor 18. The first special observation mode is a mode where a first observation image for display in which superficial blood vessels (first depth-blood vessels) are emphasized is displayed on the monitor 18. The second special observation mode is a mode where a second observation image for display in which deep blood vessels (second depth-blood vessels) are emphasized is displayed on the monitor 18. The multi-observation mode is a mode where the first and second observation images for display are automatically switched and displayed on the monitor 18. A foot switch may be used as a mode switching unit, which is used to switch a mode, other than the mode changeover SW 13a.

The processor device 16 is electrically connected to the monitor 18 and the user interface 19. The monitor 18 outputs and displays image information and the like. The user interface 19 functions as a user interface (UI) that receives an input operation, such as function settings. An external recording unit (not shown), which records image information and the like, may be connected to the processor device 16.

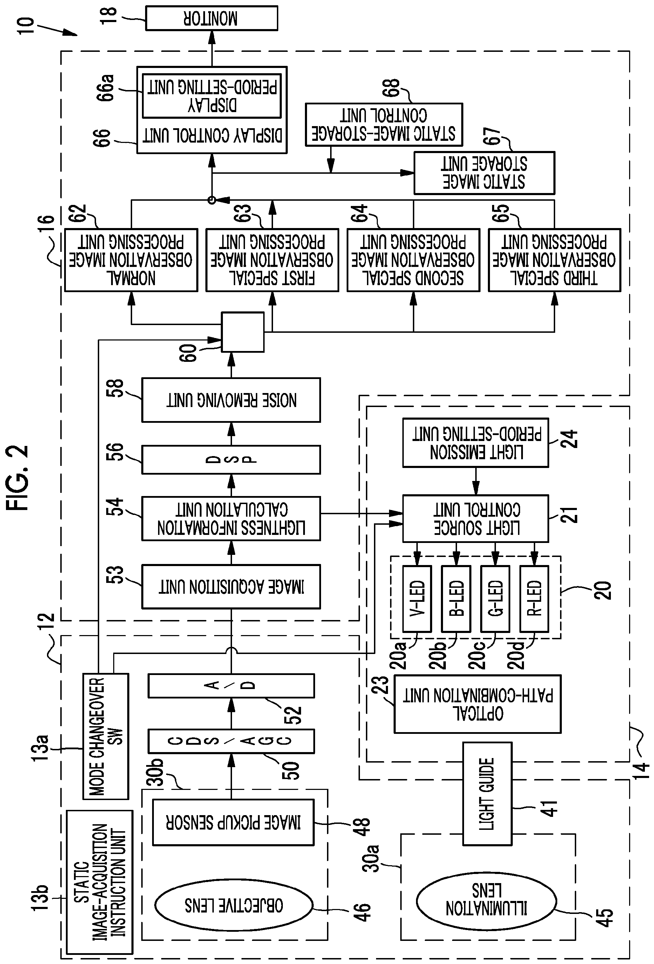

As shown in Fig. 2, the light source device 14 includes a light source unit 20, a light source control unit 21, and an optical path-combination unit 23. The light source unit 20 includes a violet light emitting diode (V-LED) 20a, a blue light emitting diode (B-LED) 20b, a green light emitting diode (G-LED) 20c, and a red light emitting diode (R-LED) 20d. The light source control unit 21 controls the drive of the LEDs 20a to 20d. The optical path-combination unit 23 combines the optical paths of four kinds of color light that are emitted from the four color LEDs 20a to 20d. The inside of an object to be examined is irradiated with the pieces of light, which are combined by the optical path-combination unit 23, through a light guide 41 inserted into the insertion part 12a and an illumination lens 45. A laser diode may be used instead of the LED.

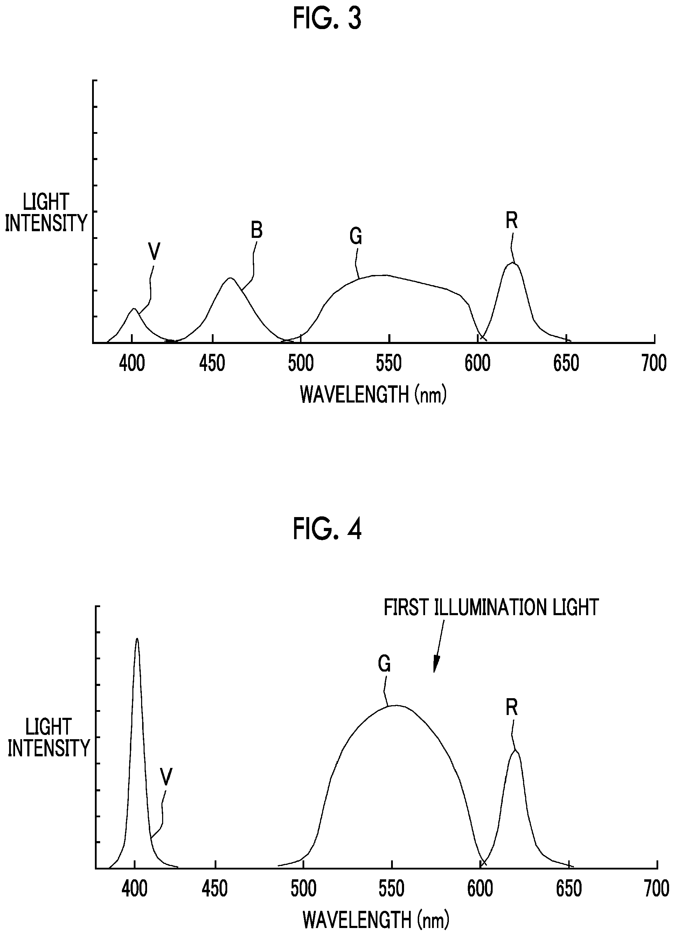

As shown in Fig. 3, the V-LED 20a generates violet light V of which the central wavelength is in the range of 405±10 nm and the wavelength range is in the range of 380 to 420 nm. The B-LED 20b generates blue light B of which the central wavelength is in the range of 460±10 nm and the wavelength range is in the range of 420 to 500 nm. The G-LED 20c generates green light G of which the wavelength range is in the range of 480 to 600 nm. The R-LED 20d generates red light R of which the central wavelength is in the range of 620 to 630 nm and the wavelength range is in the range of 600 to 650 nm. The shorter the wavelength, the shallower the depth of light penetration into the mucosa, and the shorter the wavelength, the deeper the depth of light penetration into the mucosa. Therefore, the surface layer is emphasized in an image illuminated with light having a short wavelength, and the deep layer is emphasized in an image illuminated with light having a long wavelength.

The light source control unit 21 performs control to turn on the V-LED 20a, the B-LED 20b, the G-LED 20c, and the R-LED 20d in all observation modes. Further, the light source control unit 21 controls the respective LEDs 20a to 20d so that normal light of which the light intensity ratios of violet light V, blue light B, green light G, and red light R are Vc:Bc:Gc:Rc is emitted in the normal observation mode.

Furthermore, the light source control unit 21 controls the respective LEDs 20a to 20d so that first illumination light of which the light intensity ratios of violet light V, blue light B, green light G, and red light R as first spectral information are Vs1:Bs1:Gs1:Rs1 is emitted in the first special observation mode. And the light source control unit 21 controls the respective LEDs 20a to 20d so that first illumination light of which the light intensity ratios of violet light V, blue light B, green light G, and red light R as second spectral information are Vs2:Bs2:Gs2:Rs2 is emitted in the second special observation mode.

In this specification, the light intensity ratios include a case where the ratio of at least one semiconductor light source is 0 (zero). Accordingly, the light intensity ratios include a case where any one or two or more of the respective semiconductor light sources are not turned on. For example, even though only one semiconductor light source is turned on and the other three semiconductor light sources are not turned on as in a case where the light intensity ratios of violet light V, blue light B, green light G, and red light R are 1:0:0:0, it is regarded that the light source unit 20 has light intensity ratios.

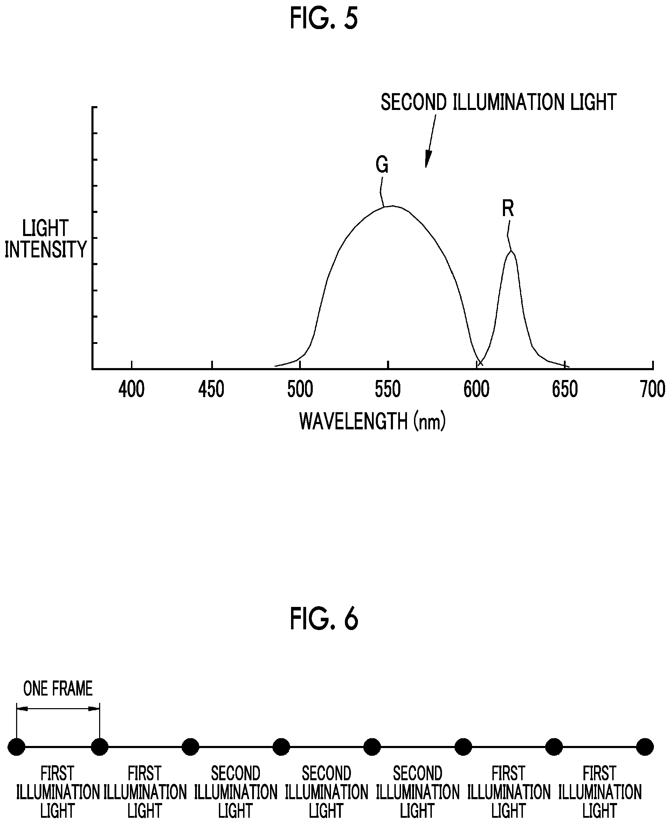

The first illumination light emphasizes superficial blood vessels, and the second illumination light emphasizes deep blood vessels. Therefore, for example, as shown in FIGS. 4 and 5, it is preferable that the first illumination light has a higher V light ratio that emphasizes the surface layer than the second illumination light.

It is preferable that both the first illumination light and the second illumination light satisfy Gs2, Rs2>0 so that the reflection on the background mucous membrane becomes large in order to accurately reproduce the color of the background mucous membrane.

Moreover, the light source control unit 21 controls the amount of illumination light to be emitted from each of the LEDs 20a to 20d on the basis of lightness information sent from a lightness information calculation unit 54 of the processor device 16.

Further, in a case where the light source control unit 21 sets the light emission period of the first illumination light to two frames and sets the light emission period of the second illumination light to three frames, the second illumination light continues to be emitted for three frames after the first illumination light continues to be emitted for two frames as shown in Fig. 6. Here, each of the light emission period of the first illumination light and the light emission period of the second illumination light is set to a period of at least two or more frames. The reason why each light emission period is set to a period of two or more frames as described above is that the illumination light of the light source device 14 is immediately switched but at least two or more frames are required to switch the image processing of the processor device 16. In addition, since there is a case where flicker occurs due to the switching of illumination light, each light emission period is set to a period of two or more frames to reduce a burden on an operator caused by flicker.

"Frame" means a unit used to control an image pickup sensor 48 that picks up the image of an object to be observed. For example, "one frame" means a period including at least an exposure period where the image pickup sensor 48 is exposed to light emitted from an object to be observed and a read-out period where image signals are read out. In this embodiment, the light emission period is determined so as to correspond to "frame" that is a unit of image pickup.

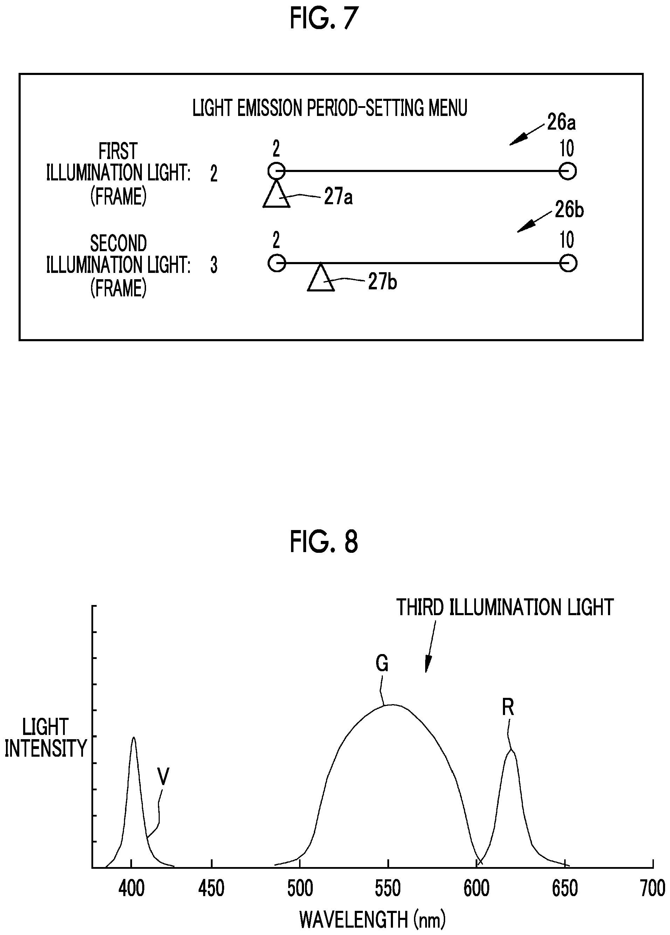

The light emission period of the first illumination light and the light emission period of the second illumination light can be appropriately changed by a light emission period-setting unit 24 that is connected to the light source control unit 21. In a case where an operation for changing a light emission period is received by the operation of the user interface 19, the light emission period-setting unit 24 displays a light emission period-setting menu shown in Fig. 7 on the monitor 18. The light emission period of the first illumination light can be changed between, for example, two frames and ten frames. Each light emission period is assigned to a slide bar 26a.

In a case where the light emission period of the first illumination light is to be changed, a user operates the user interface 19 to position a slider 27a at a position on the slide bar 26a that represents a light emission period to which the user wants to change a light emission period. Accordingly, the light emission period of the first illumination light is changed. Even in the case of the light emission period of the second illumination light, a user operates the user interface 19 to position a slider 27b at a position on a slide bar 26b (to which a light emission period in the range of, for example, two frames to ten frames is assigned) that represents a light emission period to which the user wants to change a light emission period. Accordingly, the light emission period of the second illumination light is changed.

In a case where a mode is set to the multi-observation mode, the light source control unit 21 may allow third illumination light different from the first illumination light and the second illumination light to be emitted at a timing when illumination light is switched to the second illumination light from the first illumination light or illumination light is switched to the first illumination light from the second illumination light. It is preferable that the third illumination light is emitted for at least one frame or more.

Further, it is preferable that the light intensity ratios Vs3:Bs3:Gs3:Rs3 of the third illumination light are present between the light intensity ratios Vs1:Bs1:Gs1:Rs1 of the first illumination light and the light intensity ratios Vs2:Bs2:Gs2:Rs2 of the second illumination light as third spectral information. For example, it is preferable that the light intensity ratios of the third illumination light are the averages of the light intensity ratios of the first illumination light and the light intensity ratios of the second illumination light as shown in Fig. 8. That is, Bs3 is "0", and "Vs3=(Vs1+Vs2)/2", "Gs3=(Gs1+Gs2)/2", and "Rs3=(Rs1+Rs2)/2" are satisfied with regard to the other light intensity ratios. In a case where the above-mentioned third illumination light is emitted at a timing when illumination light is switched to the first illumination light or the second illumination light, a sense of incongruity, such as a change in color caused at the time of the switching of illumination light, is not issued to a user.

As shown in Fig. 2, the light guide 41 is built in the endoscope 12 and a universal cord (a cord connecting the endoscope 12 to the light source device 14 and the processor device 16), and transmits the pieces of light, which are combined by the optical path-combination unit 23, to the distal end part 12d of the endoscope 12. A multimode fiber can be used as the light guide 41. For example, a thin fiber cable of which a total diameter of a core diameter of 105 µm, a cladding diameter of 125 µm, and a protective layer forming a covering is in the range of ϕ 0.3 to 0.5 mm can be used.

The distal end part 12d of the endoscope 12 is provided with an illumination optical system 30a and an image pickup optical system 30b. The illumination optical system 30a includes an illumination lens 45, and an object to be observed is irradiated with light transmitted from the light guide 41 through the illumination lens 45. The image pickup optical system 30b includes an objective lens 46 and an image pickup sensor 48. Light reflected from the object to be observed is incident on the image pickup sensor 48 through the objective lens 46. Accordingly, the reflected image of the object to be observed is formed on the image pickup sensor 48.

The image pickup sensor 48 is a color image pickup sensor, and picks up the reflected image of an object to be examined and outputs image signals. It is preferable that the image pickup sensor 48 is a charge coupled device (CCD) image pickup sensor, a complementary metal-oxide semiconductor (CMOS) image pickup sensor, or the like. The image pickup sensor 48 used in the invention is a color image pickup sensor that is used to obtain RGB image signals corresponding to three colors of R (red), G (green), and B (blue), that is, a so-called RGB image pickup sensor that comprises R-pixels provided with R-filters, G-pixels provided with G-filters, and B-pixels provided with B-filters.

The image pickup sensor 48 may be a so-called complementary color image pickup sensor, which comprises complementary color filters corresponding to C (cyan), M (magenta), Y (yellow), and G (green), instead of an RGB color image pickup sensor. In a case where a complementary color image pickup sensor is used, image signals corresponding to four colors of C, M, Y, and G are output. Accordingly, the image signals corresponding to four colors of C, M, Y, and G need to be converted into image signals corresponding to three colors of R, G, and B by complementary color-primary color conversion. Further, the image pickup sensor 48 may be a monochrome image pickup sensor that includes no color filter. In this case, since the light source control unit 21 causes blue light B, green light G, and red light R to be emitted in a time-sharing manner, demosaicing needs to be added to the processing of image pickup signals.

The image signals output from the image pickup sensor 48 are transmitted to a CDS/AGC circuit 50. The CDS/AGC circuit 50 performs correlated double sampling (CDS) or auto gain control (AGC) on the image signals that are analog signals. The image signals, which have been transmitted through the CDS/AGC circuit 50, are converted into digital image signals by an analog/digital converter (A/D converter) 52. The digital image signals, which have been subjected to A/D conversion, are input to the processor device 16.

The processor device 16 comprises an image acquisition unit 53, lightness information calculation unit 54, a digital signal processor (DSP) 56, a noise removing unit 58, a signal switching unit 60, a normal observation image processing unit 62, a first special observation image processing unit 63, a second special observation image processing unit 64, a display control unit 66, a static image storage unit 67, and a static image-storage control unit 68.

The image acquisition unit 53 acquires an observation image that is obtained in a case where the image of the object to be observed is picked up by in the endoscope 12. Specifically, digital color image signals obtained from the endoscope 12 are input to the image acquisition unit 53 as an observation image. The color image signals are RGB image signals that are formed of R-image signals output from the R-pixels of the image pickup sensor 48, G-image signals output from the G-pixels of the image pickup sensor 48, and B-image signals output from the B-pixels of the image pickup sensor 48. The lightness information calculation unit 54 calculates lightness information, which represents the lightness of the object to be observed, on the basis of the RGB image signals input from the image acquisition unit 53. The calculated lightness information is sent to the light source control unit 21 and is used for the control of the amount of emitted illumination light.

The DSP 56 performs various kinds of signal processing, such as defect correction processing, offset processing, gain correction processing, linear matrix processing, gamma conversion processing, and demosaicing processing, on the received image signals. Signals of defective pixels of the image pickup sensor 48 are corrected in the defect correction processing. Dark current components are removed from the RGB image signals having been subjected to the defect correction processing in the offset processing, so that an accurate zero level is set. The RGB image signals having been subjected to the offset processing are multiplied by a specific gain in the gain correction processing, so that signal levels are adjusted. The linear matrix processing for improving color reproducibility is performed on the RGB image signals having been subjected to the gain correction processing. After that, lightness or a saturation is adjusted by the gamma conversion processing. The demosaicing processing (also referred to as equalization processing or demosaicing) is performed on the RGB image signals having been subjected to the linear matrix processing, so that signals of colors deficient in each pixel are generated by interpolation. All the pixels are made to have the signals of the respective colors of R, G, and B by this demosaicing processing.

The noise removing unit 58 performs noise removal processing (for example, a moving-average method, median filtering, or the like) on the RGB image signals, which have been subjected to gamma correction and the like by the DSP 56, to remove noise from the RGB image signals. The RGB image signals from which noise has been removed are transmitted to the signal switching unit 60.

In a case where a mode is set to the normal observation mode by the mode changeover SW 13a, the signal switching unit 60 transmits the RGB image signals to the normal observation image processing unit 62. Further, in a case where a mode is set to the first special observation mode, the signal switching unit 60 transmits the RGB image signals to the first special observation image processing unit 63. Furthermore, in a case where a mode is set to the second special observation mode, the signal switching unit 60 transmits the RGB image signals to the second special observation image processing unit 64. Moreover, in a case where a mode is set to the multi-observation mode, the RGB image signals obtained from illumination using the first illumination light and image pickup are transmitted to the first special observation image processing unit 63, the RGB image signals obtained from illumination using the second illumination light and image pickup are transmitted to the second special observation image processing unit 64, and the RGB image signals obtained from illumination using the third illumination light and image pickup are transmitted to a third special observation image processing unit 65.

The normal observation image processing unit 62 performs image processing for the normal image on the RGB image signals that are obtained in the normal observation mode. The image processing for the normal image includes structure emphasis processing for the normal image and the like. The normal observation image processing unit 62 includes a parameter for a normal image, which is to be multiplied by the RGB image signals, to perform the image processing for the normal image. The RGB image signals having been subjected to the image processing for the normal image are input to the display control unit 66 from the normal observation image processing unit 62 as a normal image.

The first special observation image processing unit 63 generates the first observation image for display having been subjected to image processing (image processing for the first observation image for display), such as saturation emphasis processing, hue emphasis processing, and structure emphasis processing, on the basis of the RGB image signals of a first observation image that is obtained in a case where the object to be observed is illuminated with the first illumination light and the image thereof is picked up. In the first observation image for display, many superficial blood vessels are included and the color of the background mucous membrane is also accurately reproduced. The first special observation image processing unit 63 includes a parameter for a first observation image for display, which is to be multiplied by the RGB image signals, to perform the image processing for the first observation image for display. The first special observation image processing unit 63 does not perform superficial blood vessel emphasis processing for emphasizing superficial blood vessels so that the first observation image for display is displayed on the monitor 18 as well as possible, but may perform the superficial blood vessel emphasis processing depending on the situation of a processing load.

The second special observation image processing unit 64 generates the second observation image for display having been subjected to image processing (image processing for the second observation image for display), such as saturation emphasis processing, hue emphasis processing, and structure emphasis processing, on the basis of the RGB image signals of a second observation image that is obtained in a case where the object to be observed is illuminated with the second illumination light and the image thereof is picked up. In the second observation image for display, many deep blood vessels are included and the color of the background mucous membrane is also accurately reproduced. Since the light intensity ratios Gs1 and Rs1 of the first illumination light are the same as the light intensity ratios Gs2 and Rs2 of the second illumination light in the multi-observation mode, the color tone of at least a part of the background mucous membrane in the first observation image for display is the same as that in the second observation image for display as described above.

The second special observation image processing unit 64 includes a parameter for a second observation image for display, which is to be multiplied by the RGB image signals, to perform the image processing for the second observation image for display. The second special observation image processing unit 64 includes the same processing unit as the processing unit of the first special observation image processing unit 63, but the contents of processing of the second special observation image processing unit 64 is different from those of the first special observation image processing unit 63. The second special observation image processing unit 64 does not perform deep blood vessel emphasis processing for emphasizing deep blood vessels so that the second observation image for display is displayed on the monitor 18 as well as possible, but may perform the deep blood vessel emphasis processing depending on the situation of a processing load.

The third special observation image processing unit 65 generates a third observation image for display having been subjected to saturation emphasis processing, hue emphasis processing, and structure emphasis processing on the basis of the RGB image signals of a third observation image that is obtained in a case where the object to be observed is illuminated with the third illumination light and the image thereof is picked up. In the third observation image for display, many blood vessels of an interlayer positioned between a surface layer and a deep layer are included and the color of the background mucous membrane is also accurately reproduced. The third special observation image processing unit 65 includes a parameter for a third observation image for display, which is to be multiplied by the RGB image signals, to perform image processing for the third observation image for display. The third special observation image processing unit 65 also includes the same processing unit as the processing unit of the first special observation image processing unit 63, but the contents of processing of the third special observation image processing unit 65 is different from those of the first special observation image processing unit 63.

The display control unit 66 performs control to cause the normal image, the first observation image for display, the second observation image for display, or the third observation image for display, which is input from the normal observation image processing unit 62, the first special observation image processing unit 63, the second special observation image processing unit 64, or the third special observation image processing unit 65, to be displayed as an image that can be displayed by the monitor 18. The details of the display control unit will be described later. The static image-storage control unit 68 performs control to store an image, which is obtained according to the instruction of the static image-acquisition instruction unit 13b at the timing of a static image-acquisition instruction, in the static image storage unit 67 as a static image. In the normal observation mode, the static image-storage control unit 68 stores a normal image, which is obtained at the timing of the static image-acquisition instruction, in the static image storage unit 67 as a static image. In the first special observation mode, the static image-storage control unit 68 stores a first observation image for display, which is obtained at the timing of the static image-acquisition instruction, in the static image storage unit 67 as a static image. In the second special observation mode, the static image-storage control unit 68 stores a second observation image for display, which is obtained at the timing of the static image-acquisition instruction, in the static image storage unit 67 as a static image. Further, in the multi-observation mode, the static image-storage control unit 68 stores a set of observation image for displays, which is formed of a first observation image for display and a second observation image for display, which are obtained at the timing of the static image-acquisition instruction, in the static image storage unit 67 as static images.

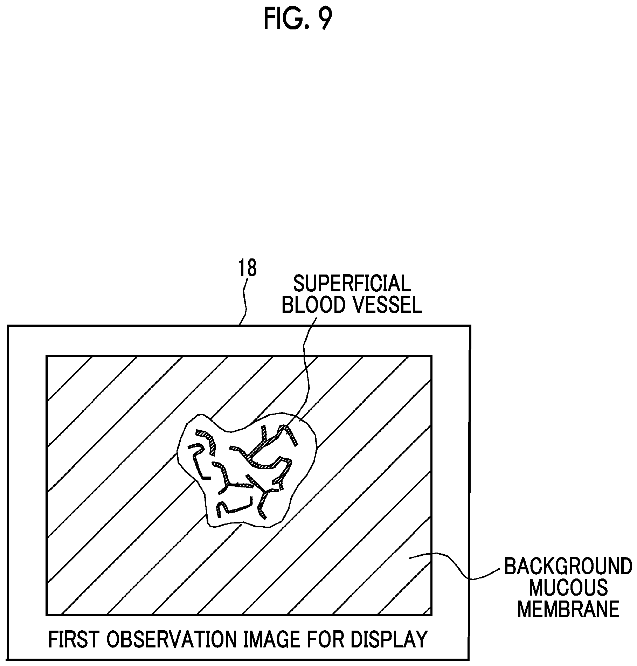

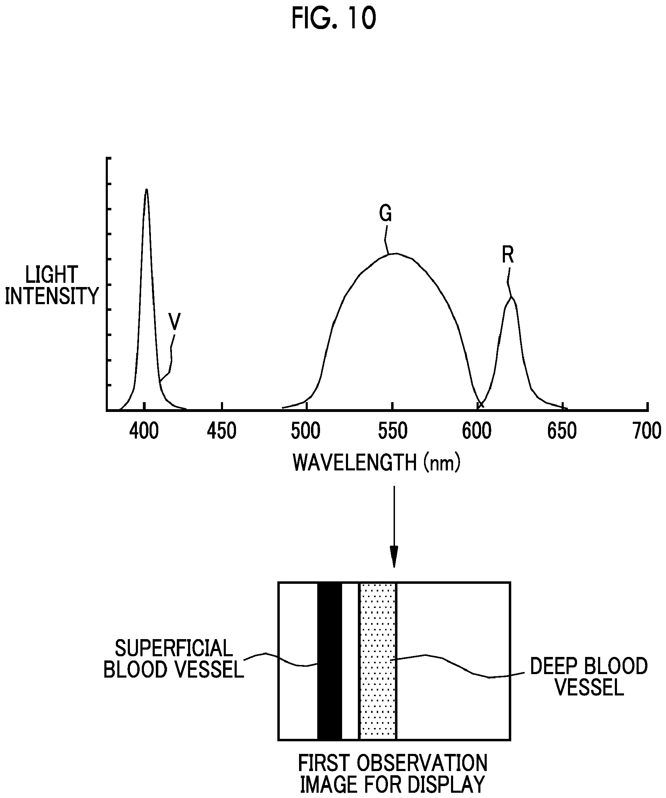

The details of the display control unit 66 will be described below. An image corresponding to each observation mode is displayed by the control of the display control unit 66. In the normal observation mode, a normal image is displayed on the monitor 18. Further, in the first special observation mode, a first observation image for display where the background mucous membrane of the object to be observed and superficial blood vessels as first specific information included in the first depth of biological tissue are displayed is displayed as shown in Fig. 9. As shown in Fig. 10, superficial blood vessels are displayed with high contrast in the first observation image for display by violet light V included in the first illumination light. Furthermore, the color tone of the background mucous membrane is maintained and accurately displayed in the first observation image for display by green light G and red light R included in the first illumination light.

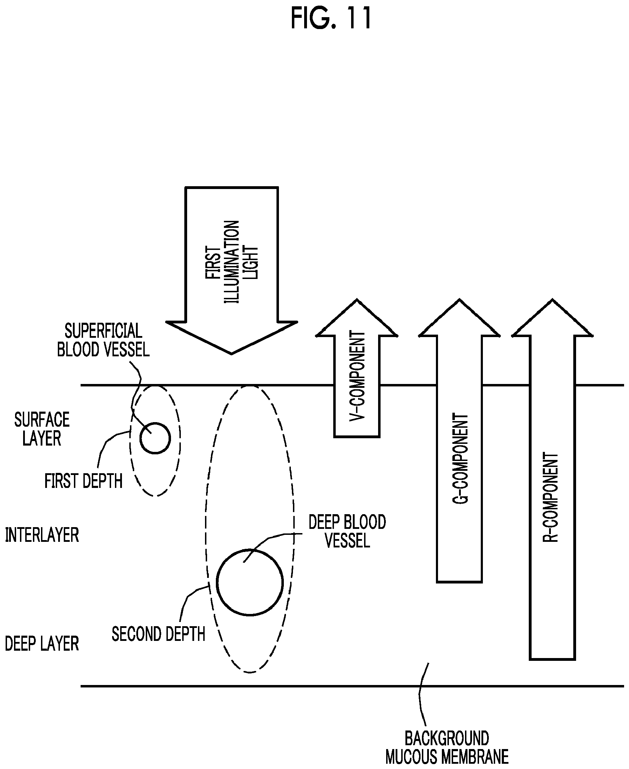

In a case where the object to be observed is illuminated with the first illumination light as shown in Fig. 11, violet light V (first depth-reaching light) of the first illumination light reaches a surface layer (first depth) and V-components of the reflected light of the first illumination light are received by the image pickup sensor 48. Accordingly, since the V-components of the reflected light are included in the first observation image for display, the contrast of superficial blood vessels is high. Further, green light G and red light R of the first illumination light reach an interlayer and a deep layer, and G-components and R-components of the reflected light of the first illumination light are received by the image pickup sensor 48. Accordingly, since the G-components of the reflected light and the R-components of the reflected light are included in the first observation image for display, the color tone of the background mucous membrane can be accurately reproduced.

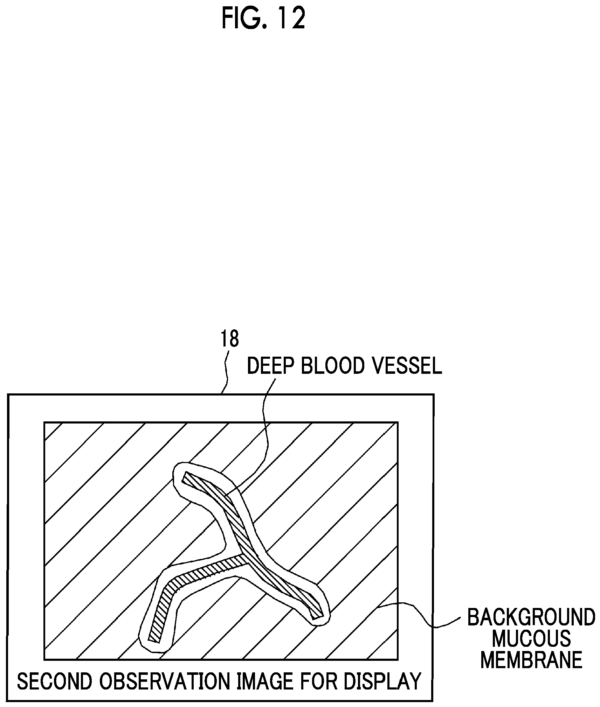

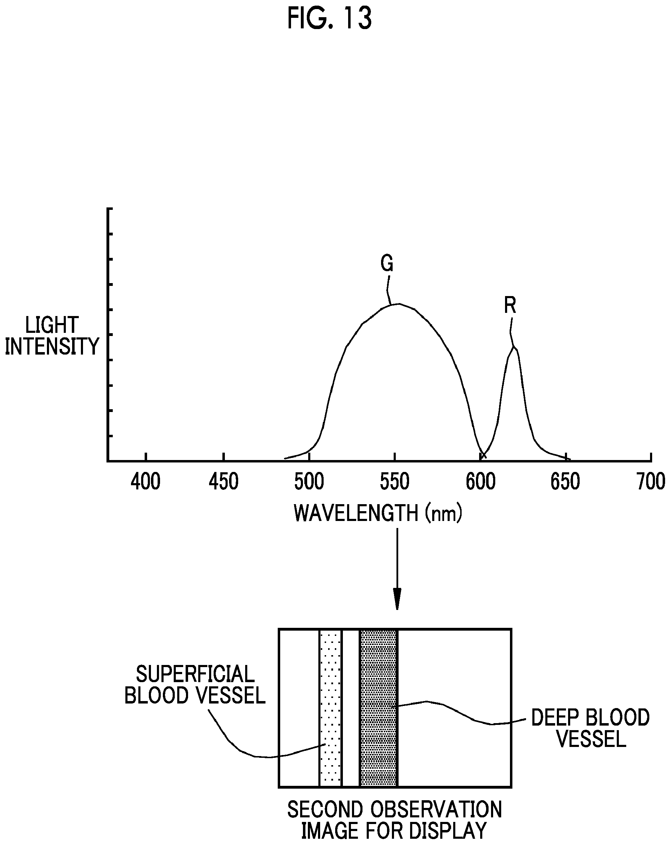

Furthermore, in the second special observation mode, a second observation image for display where the background mucous membrane of the object to be observed and deep blood vessels as second specific information included in the second depth (deeper than the first depth) of biological tissue are displayed is displayed as shown in Fig. 12. As shown in Fig. 13, superficial blood vessels and deep blood vessels are displayed in the second observation image for display by the second illumination light but the superficial blood vessels are displayed with lower contrast than those in the first observation image for display. Further, as in the first observation image for display, the color tone of the background mucous membrane is maintained and accurately displayed in the second observation image for display by green light G and red light R included in the first illumination light.

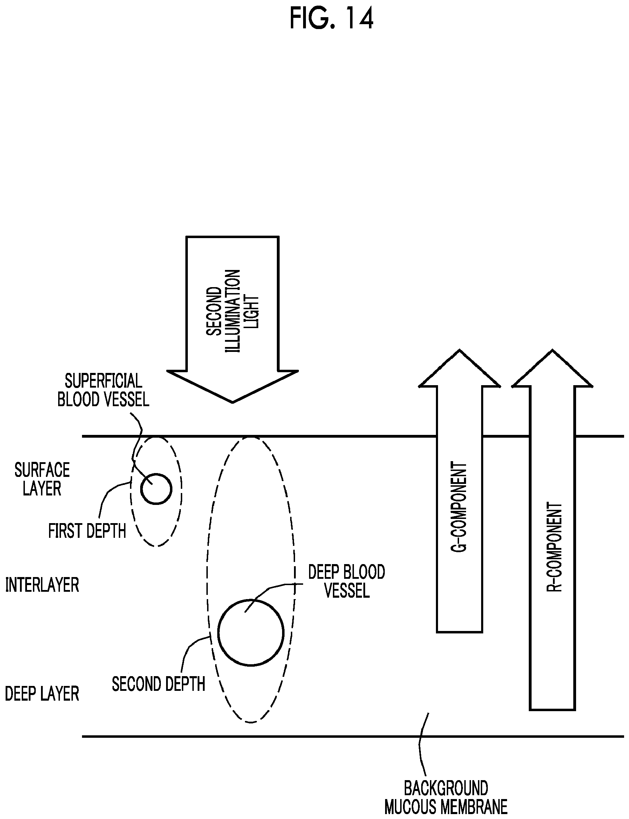

As shown in Fig. 14, green light G (second depth-reaching light) and red light R (second depth-reaching light) of the second illumination light reach an interlayer and a deep layer (second depth) and G-components and R-components of the reflected light of the second illumination light are received by the image pickup sensor 48. Accordingly, since the G-components and the R-components of the reflected light are included in the second observation image for display, the color tone of the background mucous membrane can be accurately reproduced. Superficial blood vessels and the deep blood vessels are also displayed in the second observation image for display by the G-components and the R-components of the reflected light.

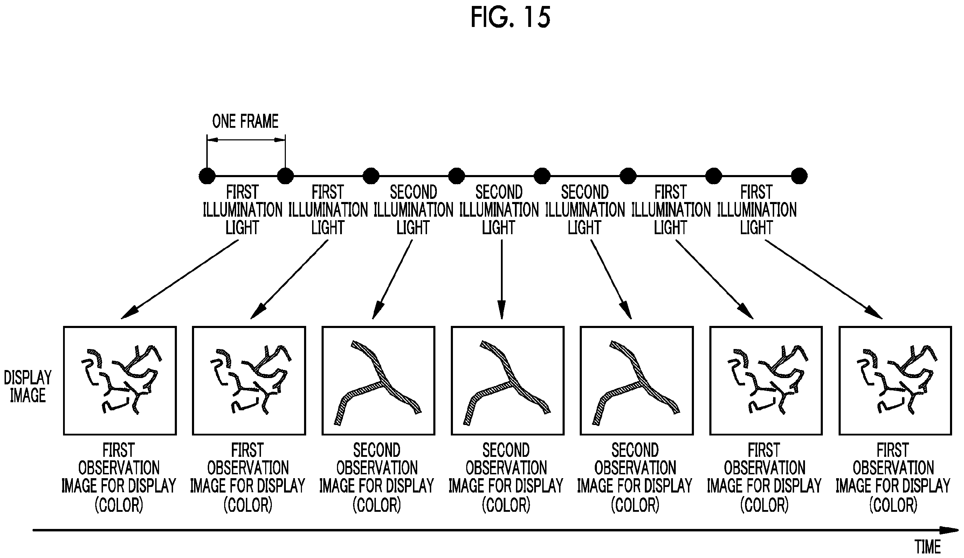

Furthermore, in the multi-observation mode, a first observation image for display and a second observation image for display, which are color images, are switched and displayed on the monitor 18 according to the light emission period of the first illumination light and the light emission period of the second illumination light as shown in Fig. 15. That is, in a case where the light emission period of the first illumination light is two frames and the light emission period of the second illumination light is three frames, the first observation image for display continues to be displayed for two frames and the second observation image for display continues to be displayed for three frames.

As described above, two kinds of the first and second observation images for display can be automatically switched and displayed in the multi-observation mode without the operation of the mode changeover SW 13a that is performed by a user. Since the first and second observation images for display are automatically switched and displayed as described above, the same object to be observed is displayed in the first and second observation images for display as long as the object to be observed is not moved or the distal end part 12d of the endoscope 12 is not moved. However, since the spectral information of the first observation image for display and the spectral information of the second observation image for display are different from each other even in the case of the same object to be observed, the object to be observed looks different depending on a difference in spectral information. That is, the visibility of superficial blood vessels is high in the first observation image for display having first spectral information, but the visibility of deep blood vessels is high in the second observation image for display having second spectral information. Accordingly, since the first and second observation images for display are switched and displayed, the visibility of a plurality of blood vessels having different depths can be improved.

Further, since each of the first and second observation images for display is an image obtained on the basis of illumination light having a red-light wavelength range, the color tone of the background mucous membrane can be reproduced. Accordingly, since the color tone of the background mucous membrane of each of the first and second observation images for display, which are displayed in the multi-observation mode, is hardly changed from that in the normal image, a sense of incongruity is not issued to a user. As a result, a user can learn about the multi-observation mode in a relatively short period. Furthermore, since the first and second observation images for display are switched and displayed, it is possible to grasp how blood vessels stand to superficial blood vessels from deep blood vessels. Further, since the color tone of the background mucous membrane of the first observation image for display is the same as that of the second observation image for display, only a difference between blood vessels can be emphasized and displayed by the switching of images.

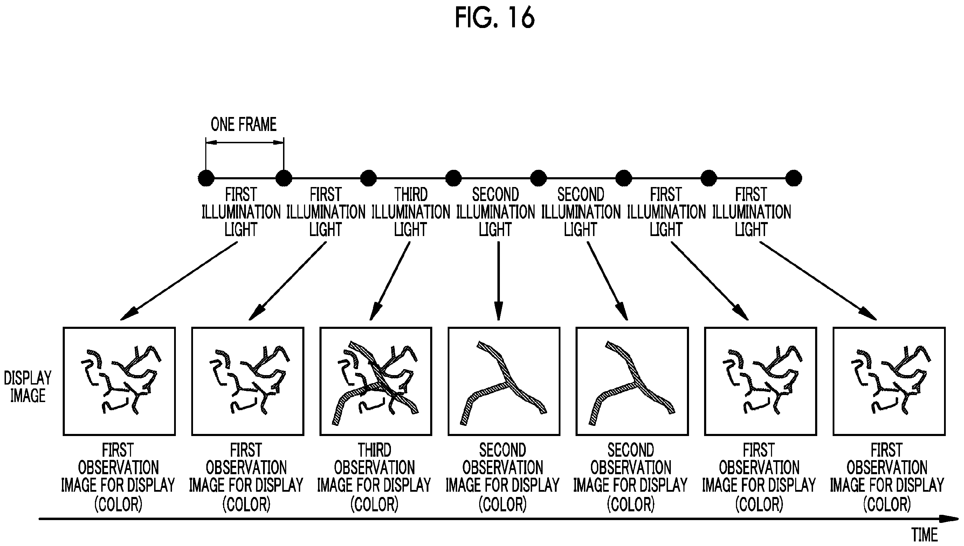

Further, in a case where illumination using the third illumination light is performed at the time of the switching of the first illumination light and the second illumination light in the multi-observation mode, the third observation image for display, which is obtained from illumination using the third illumination light and image pickup, is displayed on the monitor 18 at the time of the switching of an image to the second observation image for display from the first observation image for display as shown in Fig. 16. The blood vessels of the interlayer positioned between the surface layer and the deep layer are displayed in the third observation image for display in addition to both superficial blood vessels and deep blood vessels. Since the third observation image for display is displayed as described above, it is possible to more clearly grasp how blood vessels stand to superficial blood vessels from deep blood vessels. Furthermore, since a change in color becomes gentle by the display of the third observation image for display, a sense of incongruity to be issued to a user can be reduced.

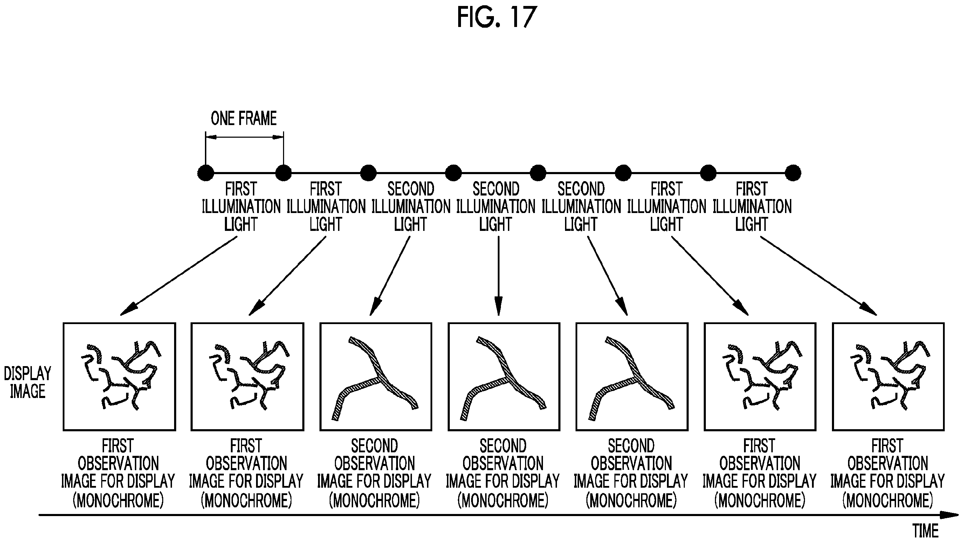

Further, the first and second observation images for display are displayed in the multi-observation mode as color images, but the first and second observation images for display may be displayed as monochrome images instead of color images as shown in Fig. 17. In a case where the first and second observation images for display as monochrome images are switched and displayed in this way, a change in color hardly occurs at portions other than blood vessels, such as superficial blood vessels and deep blood vessels. Accordingly, a user can pay attention to and observe blood vessels having different depths, such as superficial blood vessels and deep blood vessels, without feeling a sense of incongruity in the case of the switching of the first and second observation images for display.

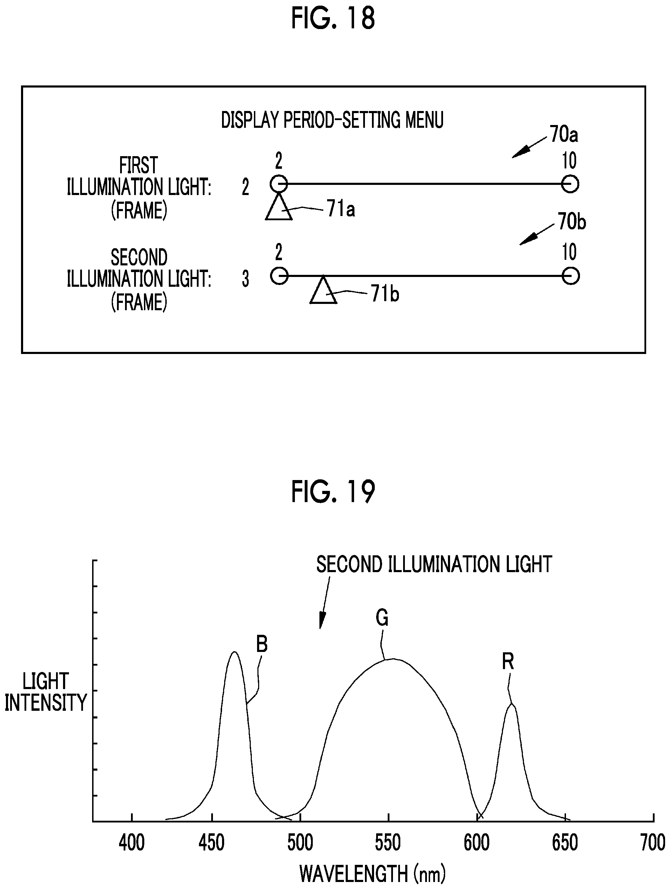

The display period of the first observation image for display and the display period of the second observation image for display can be appropriately changed by a display period-setting unit 66a that is provided in the display control unit 66. In a case where an operation for changing a display period is received by the operation of the user interface 19, the display period-setting unit 66a displays a display period-setting menu shown in Fig. 18 on the monitor 18. The display period of the first observation image for display can be changed between, for example, two frames and ten frames. Each display period is assigned to a slide bar 70a.

In a case where the display period of the first observation image for display is to be changed, a user operates the user interface 19 to position a slider 71a at a position on the slide bar 70a that represents a display period to which the user wants to change a display period. Accordingly, the display period of the first observation image for display is changed. Even in the case of the display period of the second observation image for display, a user operates the user interface 19 to position a slider 71b at a position on a slide bar 70b (to which a display period in the range of, for example, two frames to ten frames is assigned) that represents a display period to which the user wants to change a display period. Accordingly, the display period of the second observation image for display is changed.

In a case where the light emission period of the first illumination light is shorter than the display period of the first observation image for display, it is preferable that a first observation image for display corresponding to the display period is generated by complementary processing or the like so as to be displayed for the display period of the first observation image for display. In contrast, in a case where the light emission period of the second illumination light is longer than the display period of the first observation image for display, the first observation image for display may not be used for display according to the display period of the first observation image for display. Further, it is preferable that the display periods of the first and second observation images for display are set to at least two or more frames. Since the images are quickly switched in a case where the display periods of the first and second observation images for display are set to one frame, there is a concern that a user cannot recognize a difference between the first and second observation images for display.

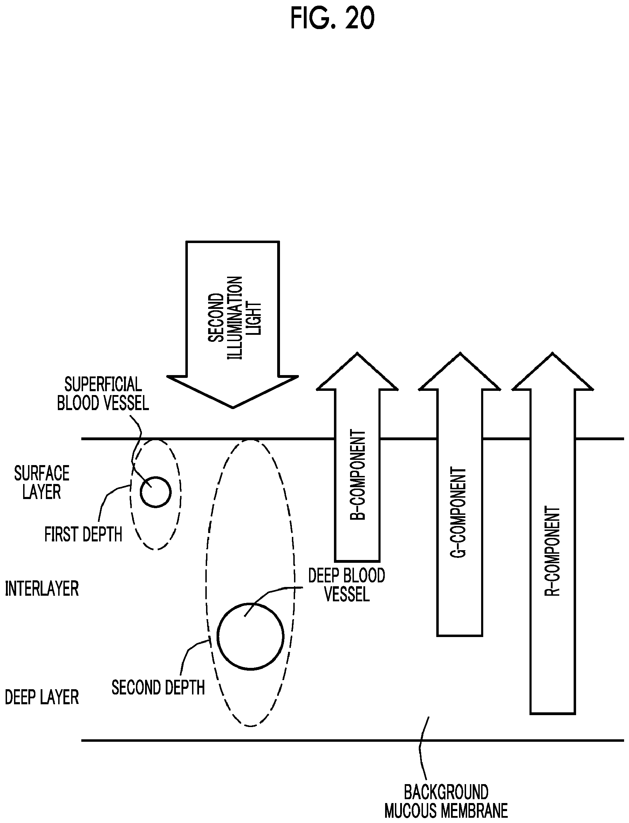

The second illumination light includes green light G and red light R in the embodiment, but may include blue light B in addition to green light G and red light R as shown in Fig. 19. As shown in Fig. 20, blue light B of the second illumination light reaches a position slightly deeper than the surface layer and B-components of the reflected light of the second illumination light are received by the image pickup sensor 48. Accordingly, superficial blood vessels can be displayed with increased contrast in the second observation image for display by the B-components of the reflected light. Since the G-components and the R-components of the reflected light are included in the second observation image for display as described above, the color tone of the background mucous membrane can be accurately reproduced.

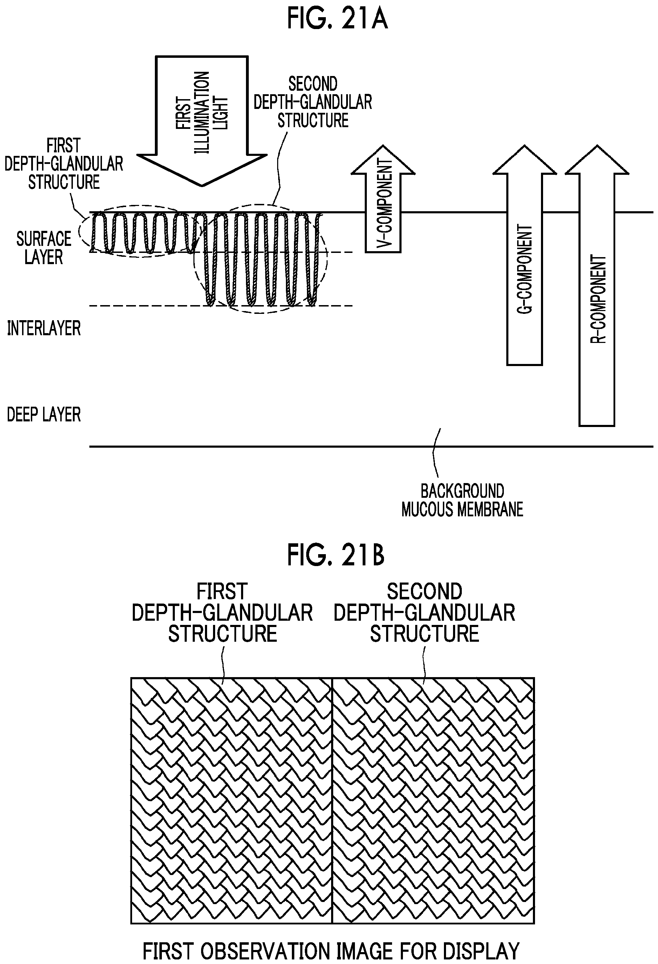

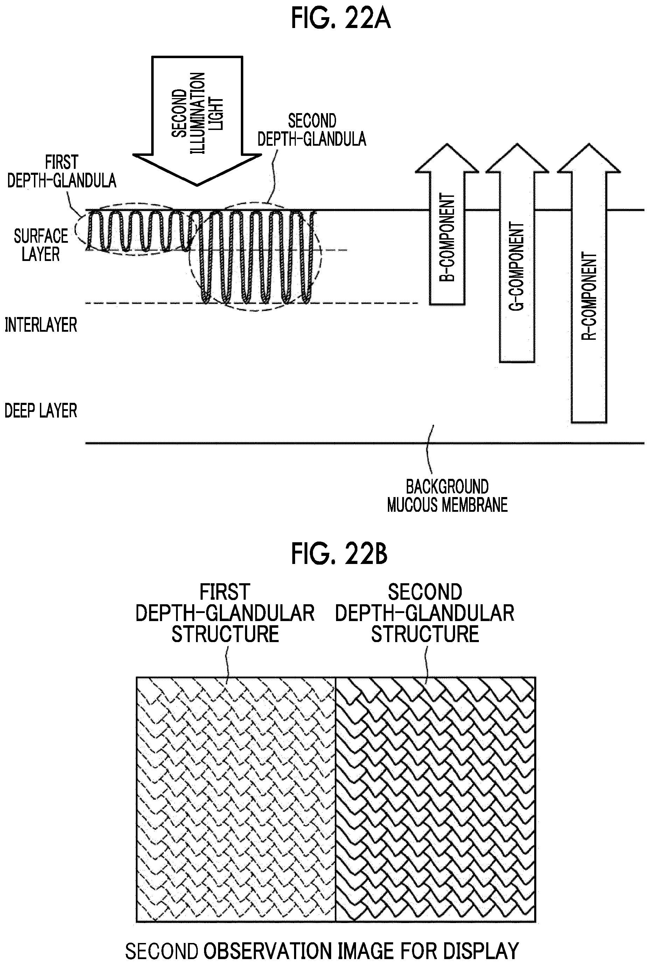

Superficial blood vessels are used as the first specific information and deep blood vessels are used as the second specific information in the embodiment, but may be used as other information. As shown in Figs. 21A and 22A, for example, first depth-glandular structures may be used as the first specific information and second depth-glandular structures deeper than the first depth may be used as the second specific information. In this case, since violet light V of the first illumination light reaches the first depth-glandular structures as shown in Fig. 21A, the first depth-glandular structures and the second depth-glandular structures are included in V-components of the reflected light of the first illumination light. Accordingly, the first depth-glandular structures and the second depth-glandular structures are displayed as the first specific information in the first observation image for display as shown in Fig. 21B. Further, in a case where the second illumination light includes blue light B, green light G, and red light R (see Fig. 19), blue light B of the second illumination light reaches the second depth-glandular structures as shown in Fig. 22A. Accordingly, B-components of the reflected light of the second illumination light include information about the first depth-glandular structures, the second depth-glandular structures, and a mucous membrane forming a layer lower than the first depth-glandular structures. Therefore, the second depth-glandular structures and the first depth-glandular structures, of which the contrast is lower than the contrast of the second depth-glandular structures, are displayed in the second observation image for display as shown in Fig. 22B. Since the layer lower than the first depth-glandular structures overlaps with the first depth-glandular structures displayed in the second observation image for display, the first depth-glandular structures displayed in the second observation image for display are displayed with lower contrast than the first depth-glandular structures displayed in the first observation image for display (or the first depth-glandular structures displayed in the second observation image for display are not seen). Since the first and second observation images for display are switched and displayed on the monitor 18 as described above, the depth (the first depth or the second depth) of the glandular structures can be recognized from a difference between the first and second observation images for display.

As a method of diagnosing whether a tumor is a cancer or not, there is a method (VS classification System) including separately analyzing a microvascular pattern (V) and a microsurface pattern (S) and diagnosing a tumor in the light of predetermined diagnostic criteria. According to this VS classification system, each of V and S is classified into regular/irregular/absent, and whether a tumor is a cancer or not is determined on the basis of the classified results. With regard to even the microsurface pattern, that is, the glandular structures, there are glandular structures having different depths as with the first depth-glandular structures and the second depth-glandular structures. Accordingly, there is a case where even glandular structures, which cannot be visually recognized using the second illumination light, can be visually recognized using the first illumination light. Therefore, blood vessels or glandular structures having different depths or heights can be visually recognized using the multi-observation mode where the first observation image for display based on the first illumination light and the second observation image for display based on the second illumination light are switched and displayed. Accordingly, the absence of, for example, glandular structures and the like can be accurately determined. As a result, the diagnostic accuracy of the VS classification system can be improved.

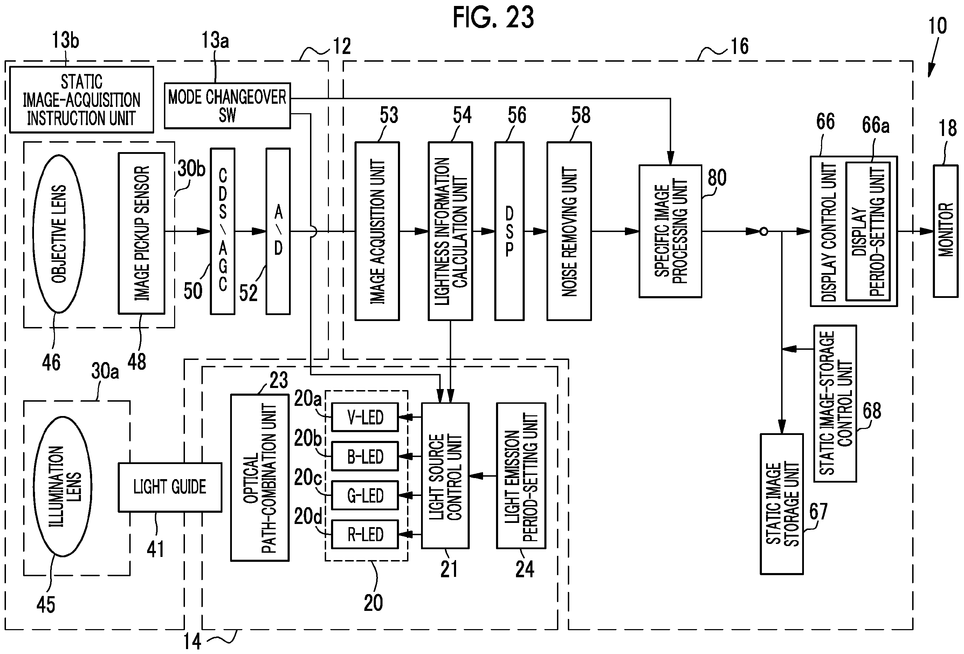

In the first embodiment, the normal observation image processing unit 62, the first special observation image processing unit 63, the second special observation image processing unit 64, and the third special observation image processing unit 65 are provided and a processing unit to be used to perform processing is determined according to an observation mode by the signal switching unit 60. However, processing may be performed by other methods. For example, a specific image processing unit 80, which is a combination of these processing units 62, 63, 64, and 65, may be provided as shown in Fig. 23 instead of the normal observation image processing unit 62, the first special observation image processing unit 63, the second special observation image processing unit 64, and the third special observation image processing unit 65; and image processing corresponding to each observation mode may be performed using a parameter corresponding to the observation mode.

For example, in the normal observation mode, the specific image processing unit 80 sets a parameter to a parameter for a normal image and performs image processing to generate a normal image. In the first special observation mode, the specific image processing unit 80 sets a parameter to a parameter for a first observation image for display and generates a first observation image for display. In the second special observation mode, the specific image processing unit 80 sets a parameter to a parameter for a second observation image for display and generates a second observation image for display. In the multi-observation mode, the specific image processing unit 80 generates each of the first and second observation images for display by switching the parameter for a first observation image for display and the parameter for a second observation image for display according to the switching of the first illumination light and the second illumination light.

### [Second embodiment]

In the first embodiment, the first illumination light used for the acquisition of the first observation image for display and the second illumination light used for the acquisition of the second observation image for display are switched and emitted to acquire two kinds of the first and second observation images for display. However, in a second embodiment, a first observation image for display having first spectral information and a second observation image for display having second spectral information different from the first spectral information are acquired from a special observation image obtained using one kind of special light. Since the first and second observation images for display of the second embodiment are generated from the special observation image of one frame, an object to be observed is the same and a deviation in position and the like do not occur between images.

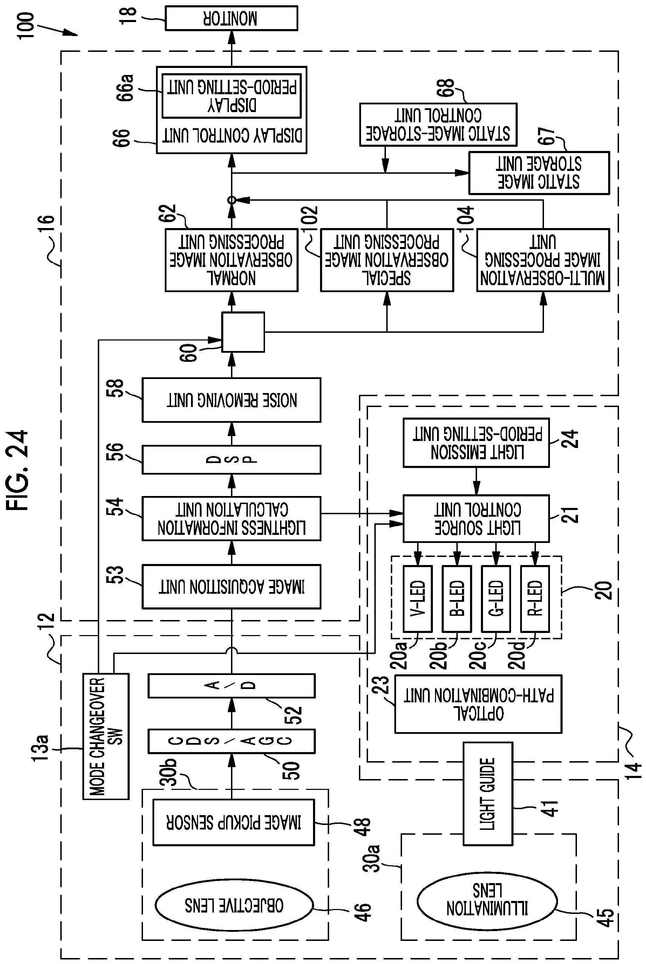

As shown in Fig. 24, an endoscope system 100 according to the second embodiment is substantially the same as an endoscope system according to the first embodiment except that a processor device 16 is provided with a special observation image processing unit 102 and a multi-observation image processing unit 104 (observation-image-for-display processing unit) instead of the first special observation image processing unit 63, the second special observation image processing unit 64, and the third special observation image processing unit 65 and has a special observation mode and a multi-observation mode instead of the first special observation mode, the second special observation mode, and the third special observation mode.

In the second embodiment, the special observation mode is a mode where a special observation image in which blood vessels having a specific depth are emphasized is displayed on the monitor 18. The multi-observation mode is a mode where a first observation image for display in which superficial blood vessels (first depth-blood vessels) are emphasized and a second observation image for display in which deep blood vessels (second depth-blood vessels) are emphasized are generated from the special observation image and the first and second observation images for display are automatically switched and displayed on the monitor 18.

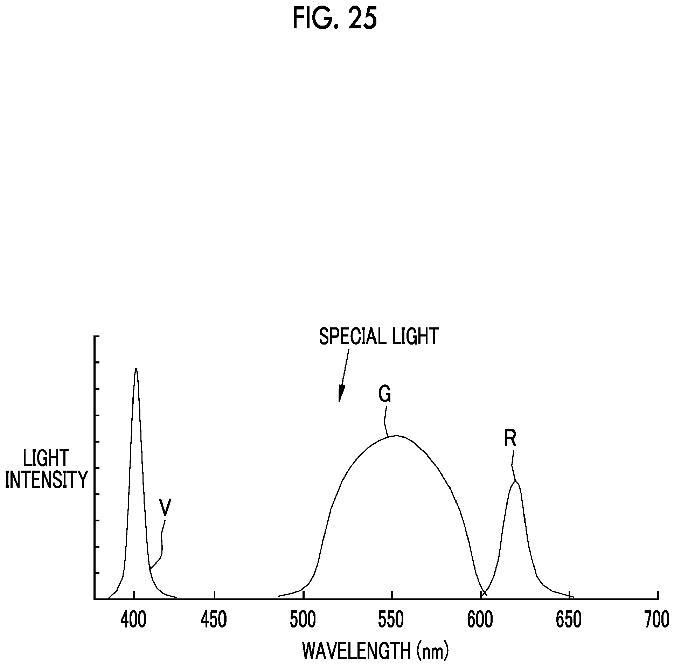

In the second embodiment, the light source control unit 21 controls the respective LEDs 20a to 20d so that special light of which the light intensity ratios of violet light V, blue light B, green light G, and red light R are Vs:Bs:Gs:Rs is emitted in the special observation mode or the multi-observation mode. It is preferable that the special light can emphasize blood vessels having a specific depth and can accurately reproduce the color of a background mucous membrane. For example, in a case where superficial blood vessels are to be emphasized as the blood vessels having a specific depth, it is preferable that Bs is set to "0" and Vs, Gs, and Rs are set to be larger than 0 as shown in Fig. 25. Since the first illumination light in this case includes violet light, green light, and red light, the first illumination light can emphasize superficial blood vessels, accurately reproduce the color of a background mucous membrane, and also emphasize various structures, such as glandular structures and unevenness.

In the second embodiment, the signal switching unit 60 transmits the RGB image signals, which have been transmitted through the noise removing unit 58, to the normal observation image processing unit 62 in a case where a mode is set to the normal observation mode by the mode changeover SW 13a. Further, in a case where a mode is set to the special observation mode, the signal switching unit 60 transmits the RGB image signals, which have been transmitted through the noise removing unit 58, to the first special observation image processing unit 63. Furthermore, in a case where a mode is set to the multi-observation mode, the signal switching unit 60 transmits the RGB image signals, which have been transmitted through the noise removing unit 58, to the multi-observation image processing unit 104.

Rs-image signals, Gs-image signals, and Bs-image signals, which are obtained in the special observation mode, are input to the first special observation image processing unit 63. Image processing for the special observation mode is performed on the Rs-image signals, the Gs-image signals, and the Bs-image signals that are input. The first special observation image processing unit 63 includes a parameter for a special observation mode, which is to be multiplied by the Rs-image signals, the Gs-image signals, and the Bs-image signal, to perform the image processing for the special observation mode. The image processing for the special observation mode includes structure emphasis processing for the special observation mode and the like. The RGB image signals having been subjected to the image processing for the special observation mode are input to the display control unit 66 from the first special observation image processing unit 63 as a special observation image.

Rs-image signals, Gs-image signals, and Bs-image signals, which are obtained in the multi-observation mode, are input to the multi-observation image processing unit 104. Image processing for the multi-observation mode is performed on the Rs-image signals, the Gs-image signals, and the Bs-image signals that are input. In the image processing for the multi-observation mode, a plurality of observation images for display in which blood vessels having depths different from each other are emphasized are generated from a special observation image of one frame. In this embodiment, the first observation image for display in which superficial blood vessels are emphasized and the second observation image for display in which deep blood vessels are emphasized are generated as the plurality of observation images for display. The details of the image processing for the multi-observation mode will be described later. The first and second observation images for display are input to the display control unit 66 from the multi-observation image processing unit 104. The multi-observation image processing unit 104 also includes a parameter for a multi-observation mode, which is to be multiplied by the Rs-image signals, the Gs-image signals, and the Bs-image signals, to perform the image processing for the multi-observation mode.

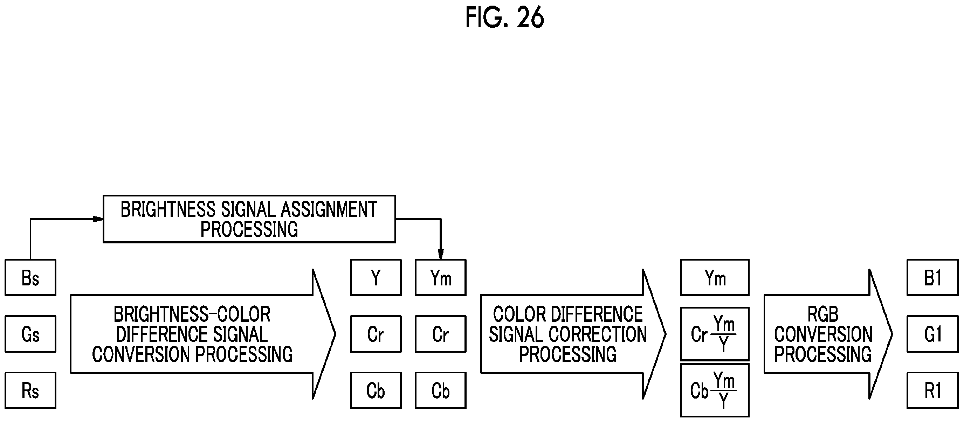

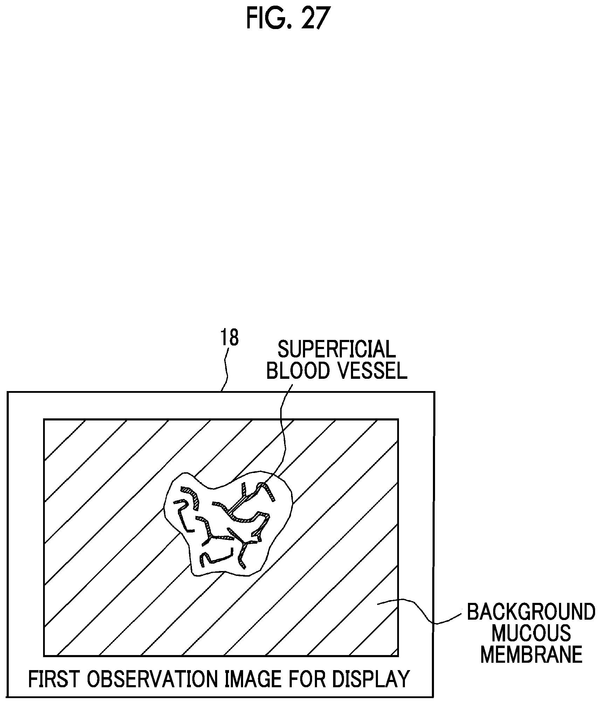

Next, the image processing for the multi-observation mode will be described. The image processing for the multi-observation mode includes first-observation-image-for-display generation processing for generating the first observation image for display and second-observation-image-for-display generation processing for generating the second observation image for display. As shown in Fig. 26, in the first-observation-image-for-display generation processing, brightness-color difference signal conversion processing is performed on the Bs-image signals, the Gs-image signals, and the Rs-image signals obtained in the multi-observation mode to convert the Bs-image signals, the Gs-image signals, and the Rs-image signals into brightness signals Y and color difference signals Cr and Cb. Then, brightness signal assignment processing for assigning the brightness signals Y to the Bs-image signals (first color signals (blue color signals) of the observation image) is performed to convert the brightness signals Y into brightness signals Ym. Since the Bs-image signals include information about superficial blood vessels as described later, an image in which superficial blood vessels are emphasized as first specific information included in a region other than the background mucous membrane as shown in Fig. 27 can be used as the first observation image for display. Further, since the first observation image for display is generated on the basis of the Gs-image signals and the Rs-image signals that include the components of green light G and red light R of the special light, the color tone of the background mucous membrane is also accurately displayed.

Next, color difference signal correction processing for correcting the deviations of the color difference signals Cr and Cb, which are caused by the conversion of the brightness signals Y into the brightness signals Ym, is performed. Specifically, the color difference signals Cr are multiplied by the converted brightness signals Ym/the converted brightness signals Y Likewise, the color difference signals Cb are multiplied by the converted brightness signals Ym/the converted brightness signals Y Accordingly, since the deviations of the color difference signals Cr and Cb are corrected, a deviation in saturation can be corrected according to the conversion of brightness while a hue is maintained (a saturation can be reduced in a case where brightness is reduced and a saturation can be increased in a case where brightness is increased). Then, RGB conversion processing is performed on the brightness signals Ym, the color difference signals Cr×Ym/Y, and the color difference signals Cb×Ym/Y, so that the brightness signals Ym, the color difference signals Cr×Ym/Y, and the color difference signals Cb×Ym/Y are converted into B1-image signals, G1-image signals, and R1-image signals. The B1-image signals, the G1-image signals, and the R1-image signals form the first observation image for display.

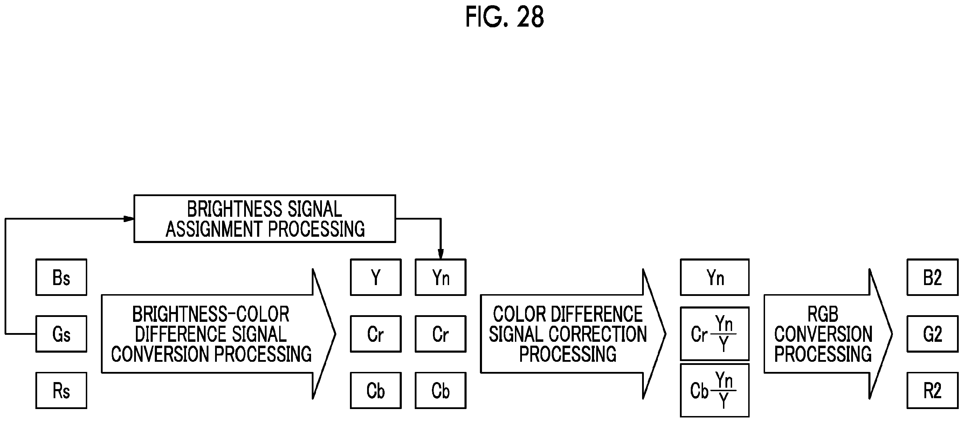

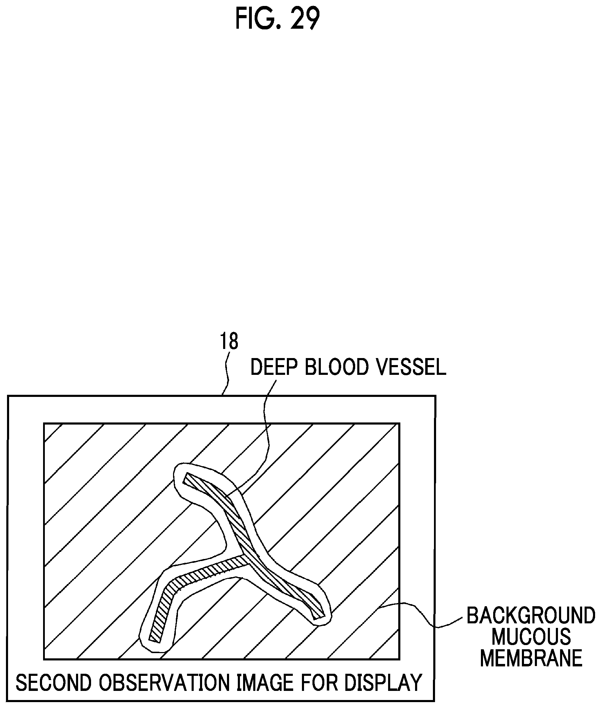

As shown in Fig. 28, in the second-observation-image-for-display generation processing, as in the first-observation-image-for-display generation processing, brightness-color difference signal conversion processing is performed on the Bs-image signals, the Gs-image signals, and the Rs-image signals obtained in the multi-observation mode to convert the Bs-image signals, the Gs-image signals, and the Rs-image signals into brightness signals Y and color difference signals Cr and Cb. Then, brightness signal assignment processing for assigning the brightness signals Y to the Gs-image signals (second color signals (green color signals) of the observation image) is performed to convert the brightness signals Y into brightness signals Yn. Since the Gs-image signals include information about deep blood vessels as described later, an image in which deep blood vessels are emphasized as second specific information included in a region other than the background mucous membrane as shown in Fig. 29 can be used as the second observation image for display. Further, since the second observation image for display is generated on the basis of the Gs-image signals and the Rs-image signals that include the components of green light G and red light R of the special light, the color tone of the background mucous membrane is also accurately displayed.

The second color signal of the observation image is a color signal that includes a component having a wavelength longer than the wavelength of the first color signal of the observation image. The first color signals are blue color signals and the second color signals are green color signals in this embodiment, but the invention is not limited thereto. For example, the first color signals may be green color signals and the second color signals may be red color signals, such as the Rs-image signals.

Next, color difference signal correction processing for correcting the deviations of the color difference signals Cr and Cb, which are caused by the conversion of the brightness signals Y into the brightness signals Yn, is performed. Specifically, the color difference signals Cr are multiplied by the converted brightness signals Yn/the converted brightness signals Y Likewise, the color difference signals Cb are multiplied by the converted color difference signals Yn/the converted color difference signals Y Accordingly, the deviations of the color difference signals Cr and Cb are corrected. RGB conversion processing is performed on the brightness signals Yn, the color difference signals Cr×Yn/Y, and the color difference signals Cb×Yn/Y, so that the brightness signals Yn, the color difference signals Cr×Yn/Y, and the color difference signals Cb×Yn/Y are converted into B2-image signals, G2-image signals, and R2-image signals. The B2-image signals, the G2-image signals, and the R2-image signals form the second observation image for display.

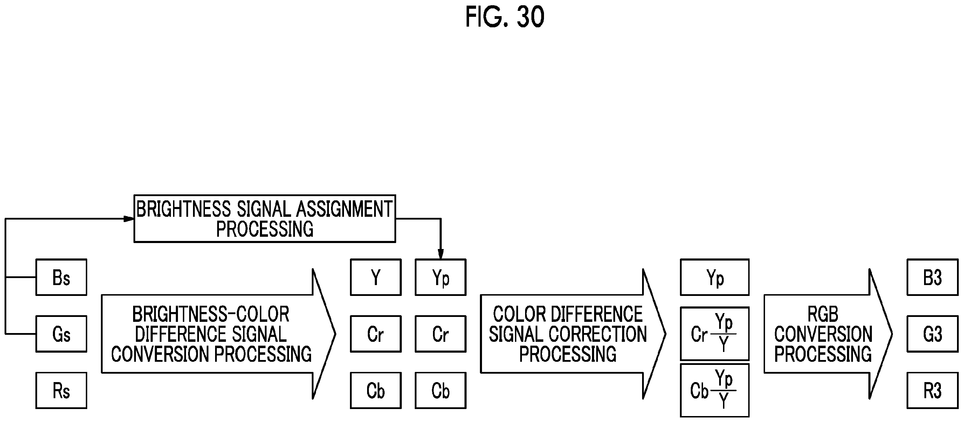

Further, a third observation image for display, which is different from the first observation image for display and the second observation image for display, may be displayed on the monitor 18 at a timing when the first and second observation images for display are switched. In this case, third-observation-image-for-display generation processing for generating the third observation image for display is performed as the image processing for the multi-observation mode. As shown in Fig. 30, in the third-observation-image-for-display generation processing, brightness-color difference signal conversion processing is performed on the Bs-image signals, the Gs-image signals, and the Rs-image signals obtained in the multi-observation mode to convert the Bs-image signals, the Gs-image signals, and the Rs-image signals into brightness signals Y and color difference signals Cr and Cb. Then, brightness signal assignment processing for assigning composite signals, which are obtained by the combination of the Bs-image signals and the Gs-image signals, to the brightness signals Y is performed to convert the brightness signals Y into brightness signal Yp. It is preferable that the composite signal is, for example, a signal ((Bs-image signal+Gs-image signal)/2) obtained by the averaging of the Bs-image signal and the Gs-image signal.

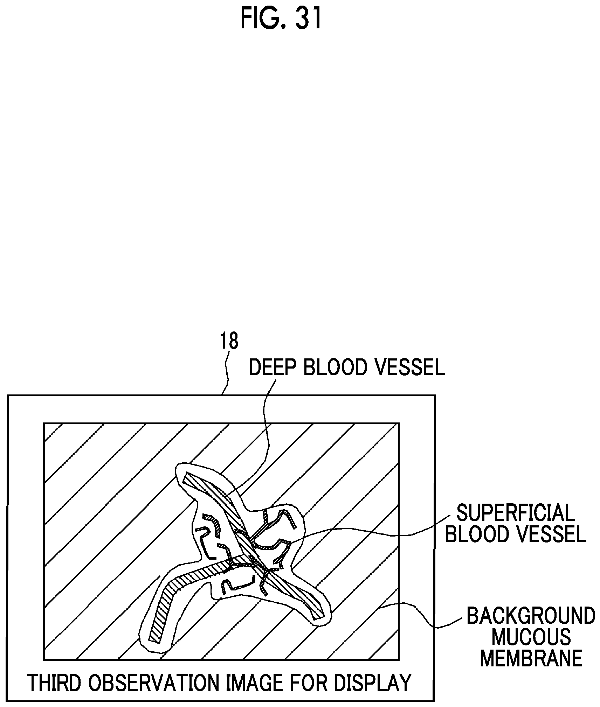

Since the composite signals include information about superficial blood vessels and deep blood vessels, an image in which superficial blood vessels and deep blood vessels are emphasized as third specific information included in a region other than the background mucous membrane as shown in Fig. 31 can be used as the third observation image for display. Further, since the third observation image for display is generated on the basis of the Gs-image signals and the Rs-image signals that include the components of green light G and red light R of the special light, the color tone of the background mucous membrane is also accurately displayed.

Next, color difference signal correction processing for correcting the deviations of the color difference signals Cr and Cb, which are caused by the conversion of the brightness signals Y into the brightness signals Yp, is performed. Specifically, the color difference signals Cr are multiplied by the converted brightness signals Yp/the converted brightness signals Y Likewise, the color difference signals Cb are multiplied by the converted brightness signals Yp/the converted brightness signals Y Accordingly, the deviations of the color difference signals Cr and Cb are corrected. Then, RGB conversion processing is performed on the brightness signals Yp, the color difference signals Cr×Yp/Y, and the color difference signals Cb×Yp/Y, so that the brightness signals Yp, the color difference signals Cr×Yp/Y, and the color difference signals Cb×Yp/Y are converted into B3-image signals, G3-image signals, and R3-image signals. The B3-image signals, the G3-image signals, and the R3-image signals form the third observation image for display.

Signals to which the Bs-image signals, the Gs-image signals, or the composite signals are to be assigned are the brightness signals Y in this embodiment, but the Bs-image signals, the Gs-image signals, or the composite signals may be assigned to other lightness information. For example, in a case where the first observation image for display is formed of lightness, a saturation, and a hue, the Bs-image signals or the Gs-image signals may be assigned to lightness corresponding to lightness information.

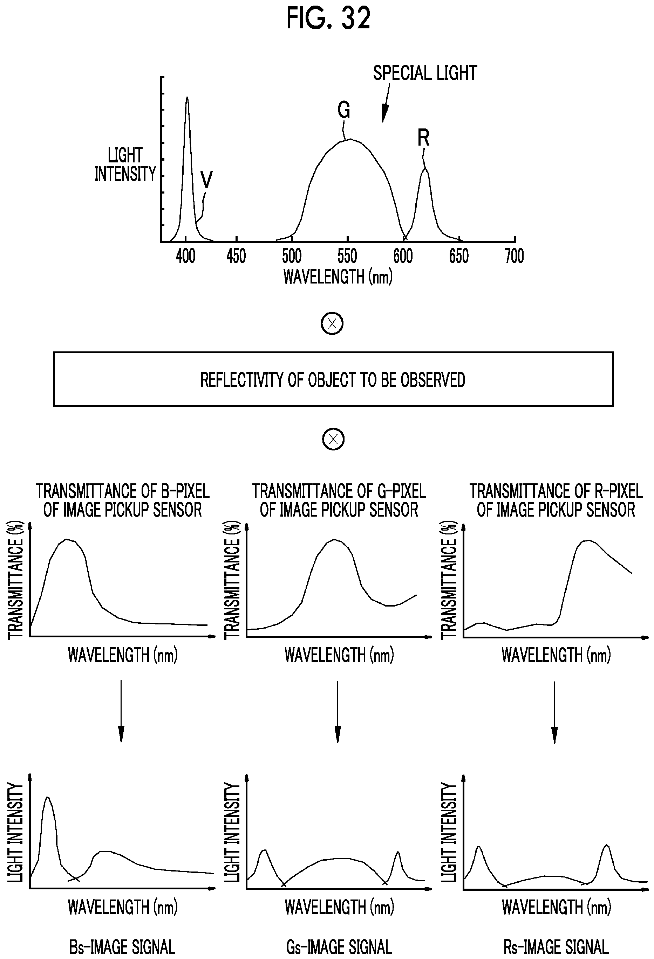

The reason why the Bs-image signals include information about superficial blood vessels and the Gs-image signals include information about deep blood vessels as described above is as follows. As shown in Fig. 32, the Bs-image signal has a signal value corresponding to light intensity that is obtained in a case where the light intensity of the special light, the reflectivity of an object to be observed, and the light transmittance of the B-pixel of the image pickup sensor 48 are multiplied together. The Bs-image signal includes many short-wavelength components of the special light. The Gs-image signal has a signal value corresponding to light intensity that is obtained in a case where the light intensity of the special light, the reflectivity of the object to be observed, and the light transmittance of the G-pixel of the image pickup sensor 48 are multiplied together. The Gs-image signal includes many medium-wavelength components of the special light. The Rs-image signal has a signal value corresponding to light intensity that is obtained in a case where the light intensity of the special light, the reflectivity of the object to be observed, and the light transmittance of the R-pixel of the image pickup sensor 48 are multiplied together. The Rs-image signal includes many long-wavelength components of the special light.

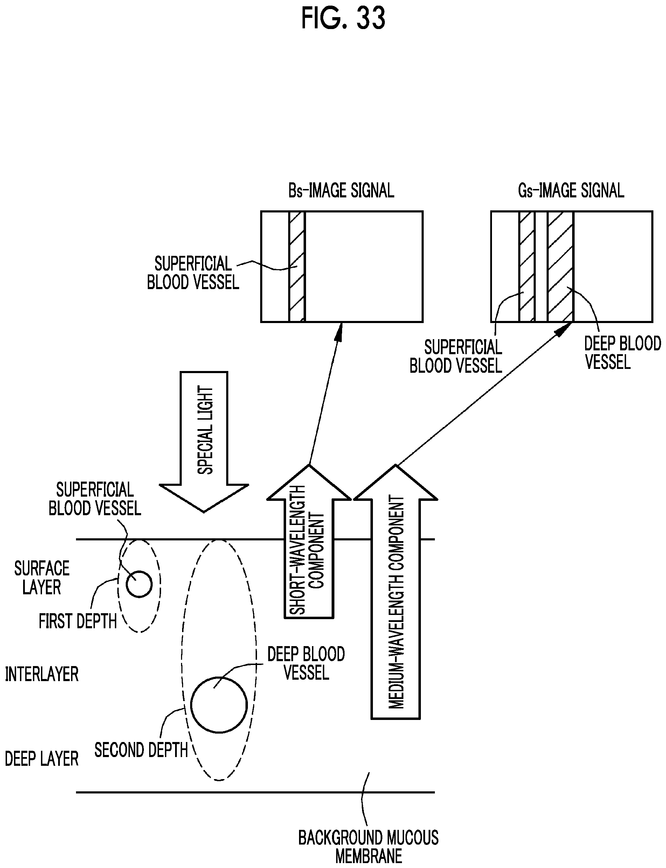

As shown in Fig. 33, many short-wavelength components (first depth-spectral information) of the special light included in the Bs-image signal correspond to components of the reflected light of light reaching the surface layer (first depth) of a mucous membrane. Accordingly, information about superficial blood vessels (first specific information) included in the surface layer of a mucous membrane is included in the Bs-image signal. On the other hand, many medium-wavelength components (second depth-spectral information) of the special light included in the Gs-image signal correspond to components of the reflected light of light reaching the vicinity (second depth) of the interlayer of a mucous membrane. Accordingly, information about superficial blood vessels (first specific information) or deep blood vessels (second specific information) included in the surface layer or the interlayer of a mucous membrane is included in the Gs-image signal. The long-wavelength components of the special light included in the Rs-image signal include information about a mucous membrane other than structures, such as blood vessels. Accordingly, information about a mucous membrane can be displayed by the Rs-image signal.

The display control unit 66 performs control to cause the monitor 18 to display the normal observation image, the special observation image, or the first or second observation image for display that is input from the normal observation image processing unit 62, the special observation image processing unit 102, or the multi-observation image processing unit 104. An image corresponding to each observation mode is displayed on the monitor 18 according to the control of the display control unit 66. In the normal observation mode, the monitor 18 displays the normal observation image. In the special observation mode, the monitor 18 displays the special observation image. In the multi-observation mode, the monitor 18 automatically switches and displays the first observation image for display or the second observation image for display according to a specific display pattern. Further, the third observation image for display may be displayed at a timing when the first and second observation images for display are switched. In a case where the third observation image for display is displayed, an object to be observed is smoothly changed according to the switching of the images without being rapidly changed.

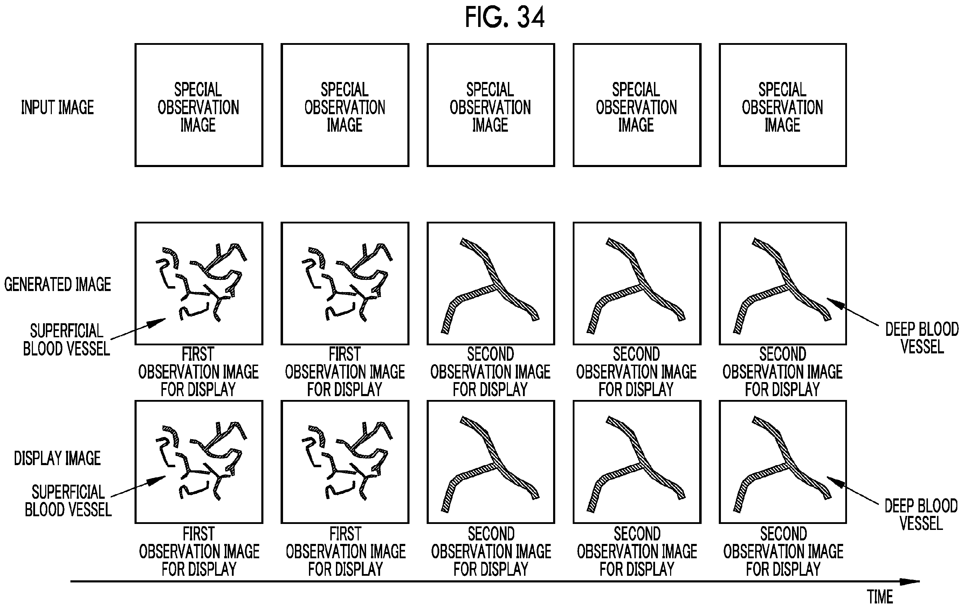

For example, in a case where the specific display pattern is to be set to a pattern where the first observation image for display is displayed for two frames and the second observation image for display is displayed for three frames in one display cycle, the following patterns are considered. As shown in Fig. 34, in a case where special observation images of two frames are input to the multi-observation image processing unit 104 as input images, the multi-observation image processing unit 104 generates first observation images for display for two frames as generated images. The generated first observation images for display for two frames are sequentially displayed on the monitor 18 as display images. After that, in a case where special observation images of three frames are input to the multi-observation image processing unit 104 as input images, the multi-observation image processing unit 104 generates second observation images for display for three frames as generated images. The generated second observation images for display for three frames are sequentially displayed on the monitor 18 as display images.

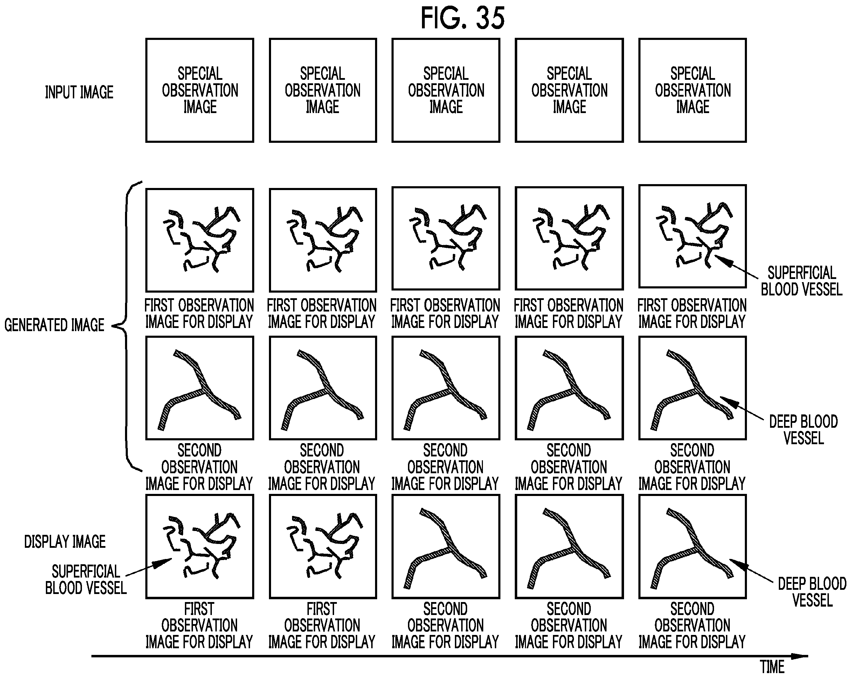

As another pattern, as shown in Fig. 35, in a case where special observation images of two frames are input to the multi-observation image processing unit 104 as input images, the multi-observation image processing unit 104 generates first and second observation images for display for two frames as generated images. Among the generated first and second observation images for display for two frames, the first observation images for display for two frames are sequentially displayed on the monitor 18 as display images. After that, in a case where special observation images of three frames are input to the multi-observation image processing unit 104 as input images, the multi-observation image processing unit 104 generates first and second observation images for display for three frames as generated images. Among the generated first and second observation images for display for three frames, the second observation images for display for three frames are sequentially displayed on the monitor 18 as display images.

According to the second embodiment, in a case where a static image-acquisition instruction is issued in the multi-observation mode by the static image-acquisition instruction unit 13b, the first and second observation images for display are generated from the special observation image obtained at the timing of the static image-acquisition instruction and these two kinds of the first observation image for display and the second observation image for display are stored in the static image storage unit 67. Accordingly, an image in which a deviation in position does not occur in the first and second observation images for display can be stored by a single static image-acquisition instruction.

It is preferable that the first and second observation images for display are switched and displayed in a display period of two or more frames. Since the images are quickly switched as described above in a case where the display periods of the first and second observation images for display are set to one frame, there is a concern that a user cannot recognize a difference between the first and second observation images for display.

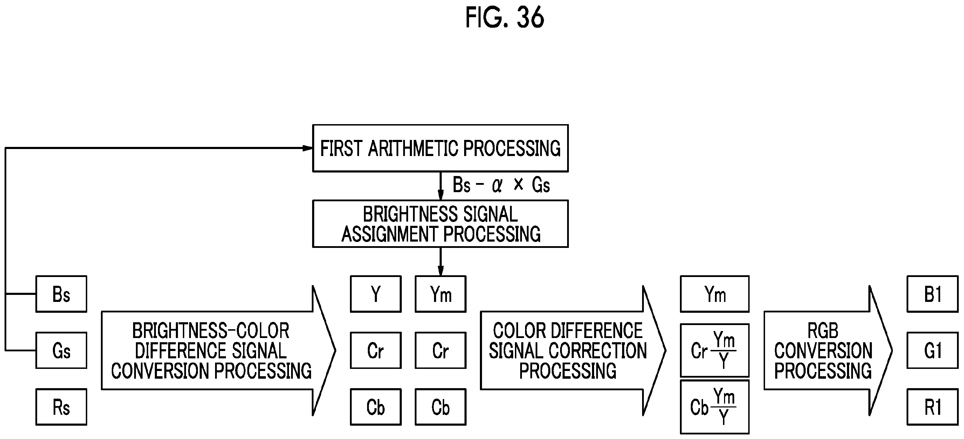

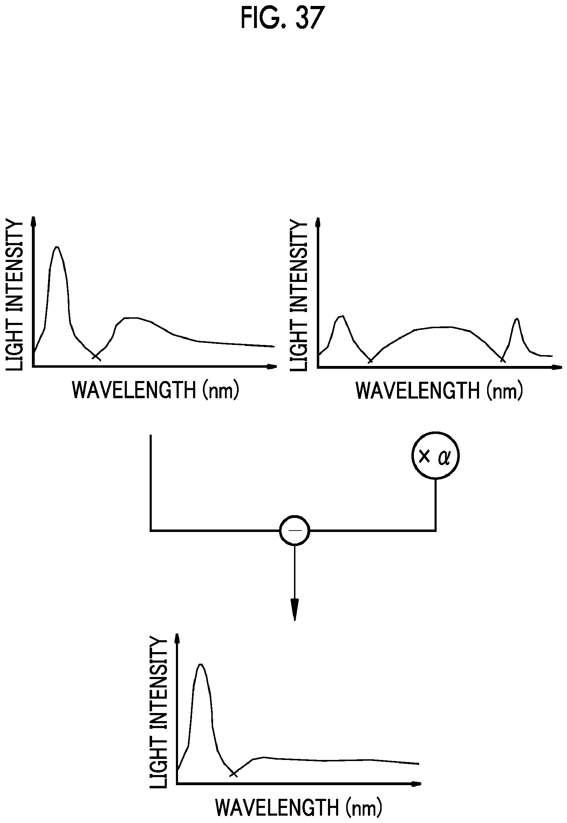

In a case where the sensitivity characteristics of the B-pixel of the image pickup sensor 48 and the sensitivity characteristics of the G-pixel of the image pickup sensor 48 partially overlap with each other, a difference between the Bs-image signal and the Gs-image signal may be small. In this case, a difference between the first and second observation images for display may be reduced. First arithmetic processing (Bs-α×Gs) for subtracting the Gs-image signal, which is multiplied by a coefficient α, from the Bs-image signal as shown in Fig. 36 is performed as one of methods of increasing a difference between the first and second observation images for display. Accordingly, the medium-wavelength components of the Gs-image signal (components of the second color signal (green color signal) of the observation image) can be removed from the Bs-image signal as shown in Fig. 37. Therefore, since information about deep blood vessels is reduced from the (Bs-α×Gs) image signal, deep blood vessels are reduced from a first observation image for display in which the (Bs-α×Gs) image signals are assigned to the brightness signals Y As a result, a difference between the first and second observation images for display can be increased. In the first arithmetic processing, deep blood vessels are reduced from the first observation image for display including superficial blood vessels (first specific information) and deep blood vessels (second specific information). Accordingly, the first arithmetic processing corresponds to "first reduction processing" of the invention.

Further, second arithmetic processing (Gs-β×Bs) for subtracting the Bs-image signal, which is multiplied by a coefficient β, from the Gs-image signal may be performed. Accordingly, the short-wavelength components of the Bs-image signal (components of the first color signal (blue color signal) of the observation image) can be removed from the Gs-image signal. Therefore, superficial blood vessels are reduced from a second observation image for display in which the (Gs-β×Bs) image signals are assigned to the brightness signals Y As a result, a difference between the first and second observation images for display can be increased. In the second arithmetic processing, superficial blood vessels are reduced from the second observation image for display including superficial blood vessels and deep blood vessels. Accordingly, the second arithmetic processing corresponds to "second reduction processing" of the invention.

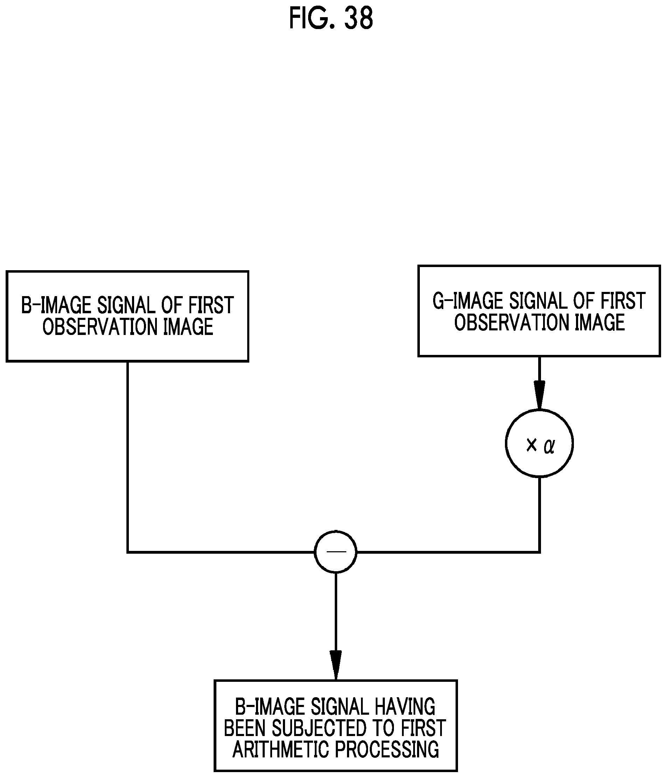

Even in the first embodiment, the first reduction processing for reducing deep blood vessels from the first observation image for display may be performed by the same method as the first arithmetic processing or processing for reducing superficial blood vessels from the second observation image for display may be performed by the same method as the second arithmetic processing. For example, as shown in Fig. 38, the first reduction processing of the first embodiment is performed by the first arithmetic processing for subtracting the G-image signal of the first observation image, which is multiplied by the coefficient α, from the B-image signal of the first observation image. Then, the first observation image for display is generated on the basis of the B-image signals of the first observation image having been subjected to the first arithmetic processing, the G-image signals of the first observation image, and the R-image signals of the first observation image. Since information about deep blood vessels of the first observation image for display is small in comparison with a case where a first observation image for display is generated without using the B-image signals having been subjected to the first arithmetic processing, the visibility of superficial blood vessels of the first observation image for display is improved.

Further, the second reduction processing of the second embodiment is performed by the second arithmetic processing for subtracting the B-image signal of the second observation image, which is multiplied by the coefficient β, from the G-image signal of the second observation image. Then, the second observation image for display is generated on the basis of the B-image signals of the second observation image, the G-image signals of the second observation image having been subjected to the second arithmetic processing, and the R-image signals of the second observation image. Since information about superficial blood vessels of the second observation image for display is small in comparison with a case where a second observation image for display is generated without using the G-image signals having been subjected to the second arithmetic processing, the visibility of deep blood vessels of the second observation image for display is improved.

In the second embodiment, the normal observation image processing unit 62, the special observation image processing unit 102, and the multi-observation image processing unit 104 are provided and a processing unit to be used to perform processing is determined according to an observation mode by the signal switching unit 60. However, processing may be performed by other methods. For example, a specific image processing unit (which is the same as the specific image processing unit 80 (see Fig. 23). not shown), which is a combination of these processing units 62, 102, and 104, may be provided instead of the normal observation image processing unit 62, the special observation image processing unit 102, and the multi-observation image processing unit 104; and image processing corresponding to each observation mode may be performed using a parameter corresponding to the observation mode.

For example, in the normal observation mode, the specific image processing unit sets a parameter to a parameter for a normal image and performs image processing to generate a normal image. In the first special observation mode, the specific image processing unit sets a parameter to a parameter for a first observation image for display and generates a first observation image for display. In the second special observation mode, the specific image processing unit sets a parameter to a parameter for a second observation image for display and generates a second observation image for display. In the multi-observation mode, the specific image processing unit generates each of the first and second observation images for display by switching the parameter for a first observation image for display and the parameter for a second observation image for display according to the display switching of a predetermined first observation image for display and a predetermined second observation image for display.

In the first and second embodiments, special processing for improving the visibility of blood vessels, such as superficial blood vessels or deep blood vessels, is not performed on the first observation image for display or the second observation image for display to reduce the processing load of the processor device 16. However, special processing for improving the visibility of the blood vessels may be performed depending on a situation.

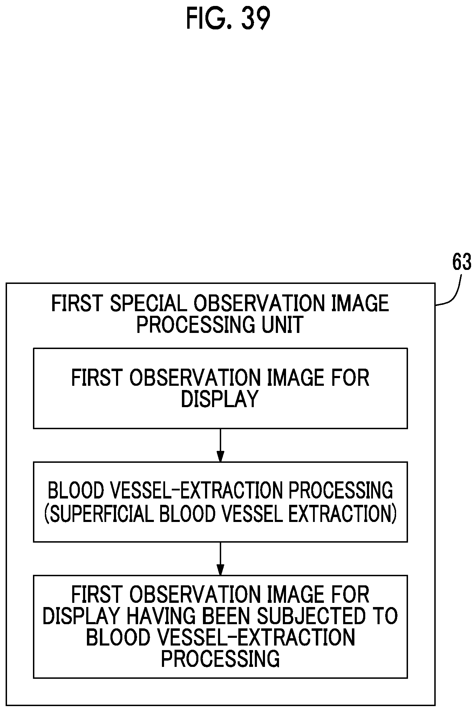

For example, in the case of the first embodiment, the first special observation image processing unit 63 performs blood vessel-extraction processing for extracting superficial blood vessels on the first observation image as shown in Fig. 39. The superficial blood vessels extracted by the blood vessel-extraction processing are superimposed and displayed on the first observation image for display. Likewise, the second special observation image processing unit 64 performs blood vessel-extraction processing for extracting deep blood vessels on the second observation image. The deep blood vessels extracted by the blood vessel-extraction processing are superimposed and displayed on the second observation image for display. In a case where the blood vessels extracted by the blood vessel-extraction processing are displayed as described above, the visibility of the blood vessels on an image is improved in comparison with a case where the extracted blood vessels are not displayed. In a case where the third observation image for display is to be displayed, the third special observation image processing unit 65 performs blood vessel-extraction processing for extracting superficial blood vessels or deep blood vessels on the third observation image for display. The superficial blood vessels or the deep blood vessels extracted by the blood vessel-extraction processing are displayed in the third observation image for display.

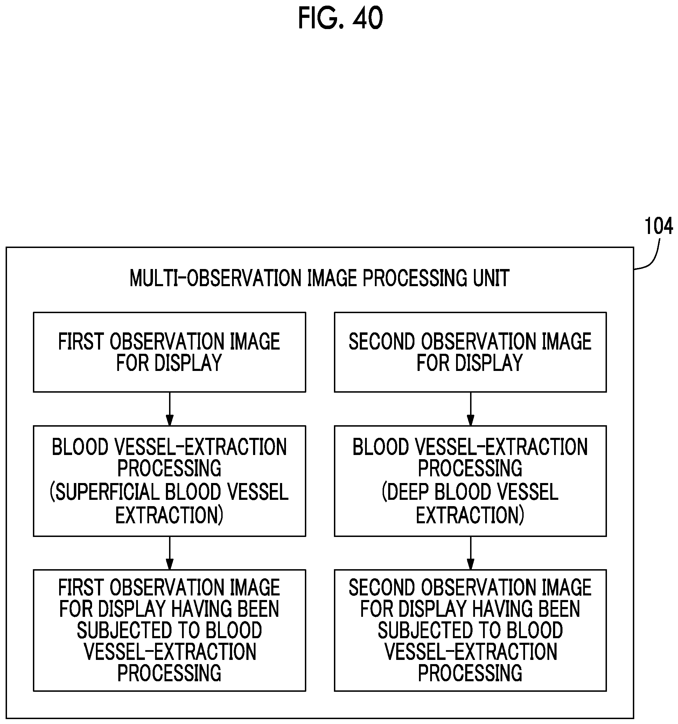

Further, in the case of the second embodiment, as shown in Fig. 40, the multi-observation image processing unit 104 performs blood vessel-extraction processing for extracting superficial blood vessels and blood vessel-extraction processing for extracting deep blood vessels on the special observation image. Then, the superficial blood vessels extracted by the blood vessel-extraction processing are superimposed and displayed on the first observation image for display. Further, the deep blood vessels extracted by the blood vessel-extraction processing are superimposed and displayed on the second observation image for display. In a case where the blood vessels extracted by the blood vessel-extraction processing are displayed as described above, the visibility of the blood vessels on an image is improved in comparison with a case where the extracted blood vessels are not displayed. In a case where the third observation image for display is to be displayed, the multi-observation image processing unit 104 performs blood vessel-extraction processing for extracting superficial blood vessels or deep blood vessels on the third observation image for display. The superficial blood vessels or the deep blood vessels extracted by the blood vessel-extraction processing are displayed in the third observation image for display.

The hardware structures of the processing units, which are included in the processor device 16 in the first and second embodiments, such as the image acquisition unit 53, the lightness information calculation unit 54, the DSP 56, the noise removing unit 58, the normal observation image processing unit 62, the first special observation image processing unit 63, the second special observation image processing unit 64, the third special observation image processing unit 65, the static image storage unit 67, the display control unit 66, the display period-setting unit 66a, the static image-storage control unit 68, the specific image processing unit 80, the special observation image processing unit 102, and the multi-observation image processing unit 104, are various processors to be described below. The various processors include: a central processing unit (CPU) that is a general-purpose processor functioning as various processing units by executing software (program); a programmable logic device (PLD) that is a processor of which circuit configuration can be changed after manufacture, such as a field programmable gate array (FPGA); a dedicated electrical circuit that is a processor having circuit configuration designed exclusively to perform various kinds of processing; and the like.

One processing unit may be formed of one of these various processors, or may be formed of a combination of two or more same kind or different kinds of processors (for example, a plurality of FPGAs or a combination of a CPU and an FPGA). Further, a plurality of processing units may be formed of one processor. As an example where a plurality of processing units are formed of one processor, first, there is an aspect where one processor is formed of a combination of one or more CPUs and software as typified by a computer, such as a client or a server, and functions as a plurality of processing units. Second, there is an aspect where a processor fulfilling the functions of the entire system, which includes a plurality of processing units, by one integrated circuit (IC) chip as typified by System On Chip (SoC) or the like is used. In this way, various processing units are formed using one or more of the above-mentioned various processors as hardware structures.

In addition, the hardware structures of these various processors are more specifically electrical circuitry where circuit elements, such as semiconductor elements, are combined.

The invention can be applied to various medical image processing devices other than the processor device that is to be combined with the endoscope systems described in the first and second embodiments.

### Explanation of References

10: endoscope system
12: endoscope
12a: insertion part
12b: operation part
12c: bendable part
12d: distal end part
12e: angle knob
13b: static image-acquisition instruction unit
14: light source device
16: processor device
18: monitor
19: user interface
20: light source unit
20a: V-LED (Violet Light Emitting Diode)
20b: B-LED (Blue Light Emitting Diode)
20c: G-LED (Green Light Emitting Diode)
20d: R-LED (Red Light Emitting Diode)
21: light source control unit
23: optical path-combination unit
24: light emission period-setting unit
26a: slide bar
26b: slide bar
27a: slider
27b: slider
30a: illumination optical system
30b: image pickup optical system
41: light guide
45: illumination lens
46: objective lens
48: image pickup sensor
50: CDS/AGC circuit
53: image acquisition unit
54: lightness information calculation unit
56: DSP (Digital Signal Processor)
58: noise removing unit
60: signal switching unit
62: normal observation image processing unit
63: first special observation image processing unit
64: second special observation image processing unit
65: third special observation image processing unit
66: display control unit
66a: display period-setting unit
67: static image storage unit
68: static image-storage control unit
70a: slide bar
70b: slide bar
71a: slider
71b: slider
80: specific image processing unit
100: endoscope system
102: special observation image processing unit
104: multi-observation image processing unit

## Claims

1. An endoscope system comprising:
an image acquisition unit (53) configured to acquire an observation image;
a display control unit (66) configured to cause a display unit to automatically switch and display at least two first and second observation images acquired by the image acquisition unit (66) for display, in which the same object to be observed is displayed, for a display period of at least two or more frames,
wherein first spectral information of the first observation image for display and second spectral information of the second observation image for display are different from each other;
a light source unit (20) configured to emit first illumination light having the first spectral information and second illumination light having the second spectral information;
a light source control unit (21) configured to cause each of the first illumination light and the second illumination light to be emitted for a light emission period and automatically switches the first illumination light and the second illumination light,
wherein the first observation image for display is obtained in a case where an image of the object to be observed illuminated with the first illumination light is picked up;
the second observation image for display is obtained in a case where an image of the object to be observed illuminated with the second illumination light is picked up;
**characterized in that**
the light emission period is at least two or more frames,
wherein the light source control unit (21) is configured to make
the color tone of at least a part of a background mucous membrane of the first observation image for display be the same as the color tone of at least a part of a background mucous membrane of the second observation image for display by making light intensity ratios of green light and red light in the first illumination light be the same as light intensity ratios of green light and red light in the second illumination light.

2. The endoscope system according to claim 1,
wherein the display control unit (66) is configured to cause the display unit to display a third observation image for display different from the first observation image for display and the second observation image for display at a timing when the first observation image for display and the second observation image for display are switched.

3. The endoscope system according to claim 2,
wherein the light source control unit (21) is configured to cause third illumination light different from the first illumination light and the second illumination light to be emitted at a timing when the first observation image for display and the second observation image for display are switched.

4. The endoscope system according to any of the preceding claims,
wherein the first illumination light includes violet light, green light, and red light, and
the second illumination light includes green light and red light.

5. The endoscope system according to any one of the preceding claims,
wherein the first illumination light includes violet light, green light, and red light, and
the second illumination light includes blue light, green light, and red light.

6. The endoscope system according to any one of the preceding claims,
wherein the first observation image for display includes first specific information, which is included in a first depth of biological tissue, in a region thereof other than the background mucous membrane,
the second observation image for display includes second specific information, which is included in a second depth of the biological tissue, in a region thereof other than the background mucous membrane, and
the second depth is deeper than the first depth.

7. The endoscope system according to claim 6,
wherein the first illumination light includes first depth-reaching light reaching the first depth, and the second illumination light includes second depth-reaching light reaching the second depth.

8. The endoscope system according to claim 1, further comprising:
an observation-image-for-display processing unit configured to generate an observation image for display on the basis of the observation image,
wherein the observation-image-for-display processing unit is configured to assign a first color signal of the observation image to lightness information to generate the first observation image for display as the observation image for display, and to assign a second color signal of the observation image to lightness information to generate the second observation image for display as the observation image for display.

9. The endoscope system according to claim 8,
wherein the observation image is obtained in a case where an image of the object to be observed illuminated with special light including violet light, green light, and red light is picked up.

10. The endoscope system according to claim 8 or 9,
wherein the display control unit (66) is configured to cause the display unit to display a third observation image for display different from the first observation image for display and the second observation image for display at a timing when the first observation image for display and the second observation image for display are switched.

11. The endoscope system according to claim 10,
wherein the observation-image-for-display processing unit is configured to assign a composite signal, which is obtained by combination of the first color signal and the second color signal of the observation image, to lightness information to generate the third observation image for display at a timing when the first observation image for display and the second observation image for display are switched.

12. The endoscope system according to any one of claims 8 to 11,
wherein the first observation image for display includes first specific information, which is included in a first depth of biological tissue, in a region thereof other than the background mucous membrane,
the second observation image for display includes second specific information, which is included in a second depth of the biological tissue, in a region thereof other than the background mucous membrane, and
the second depth is deeper than the first depth.

13. The endoscope system according to claim 12,
wherein the first spectral information includes first depth-spectral information corresponding to the first depth and the second spectral information includes second depth-spectral information corresponding to the second depth.

14. The endoscope system according to any one of claims 1 to 13,
wherein a parameter for the first observation image for display used for the generation of the first observation image for display and a parameter for the second observation image for display used for the generation of the second observation image for display are switched according to switching of the first observation image for display and the second observation image for display.

15. The endoscope system according to any one of claims 1 to 5, 8 to 11, and 14,
wherein the first observation image for display includes first specific information, which is included in a first depth of biological tissue, in a region thereof other than the background mucous membrane and the second observation image for display includes second specific information, which is included in a second depth of the biological tissue deeper than the first depth, in a region thereof other than the background mucous membrane, and
first reduction processing for reducing the second specific information from the first observation image for display or second reduction processing for reducing the first specific information from the second observation image for display is performed.

16. The endoscope system according to any one of claims 1 to 15,
wherein the display control unit (66) includes a display period-setting unit (66a) that sets a display period of the first observation image for display and a display period of the second observation image for display.

17. The endoscope system according to any one of claims 1 to 16, further comprising:
a static image-acquisition instruction unit (13b) is configured to issue a static image-acquisition instruction; and
a static image-storage control unit (68) is configured to perform control to store a set of observation images for display, which includes the first observation image for display and the second observation image for display, in a static image storage unit (67) in a case where the static image-acquisition instruction is issued.

18. The endoscope system according to any one of claims 6, 7, 12, 13, and 15,
wherein the first specific information is first depth-blood vessels positioned at the first depth, and the second specific information is second depth-blood vessels positioned at the second depth.

19. The endoscope system according to claim 18,
wherein the first depth-blood vessels extracted by blood vessel-extraction processing are displayed in the first observation image for display, and the second depth-blood vessels extracted by the blood vessel-extraction processing are displayed in the second observation image for display.

20. The endoscope system according to any one of claims 6, 7, 12, 13, and 15,
wherein the first specific information is first depth-glandular structures positioned at the first depth, and the second specific information is second depth-glandular structures positioned at the second depth.

## Patentansprüche

1. Endoskopsystem, umfassend:
eine Bilderfassungseinheit (53), die so konfiguriert ist, dass sie ein Beobachtungsbild erfasst;
eine Anzeigesteuereinheit (66), die so konfiguriert ist, dass sie eine Anzeigeeinheit veranlasst, automatisch umzuschalten und mindestens zwei erste und zweite Beobachtungsbilder, die von der Bilderfassungseinheit (66) zur Anzeige erfasst wurden, in denen das gleiche zu beobachtende Objekt angezeigt wird, für einen Anzeigezeitraum von mindestens zwei oder mehr Einzelbildern anzuzeigen,
wobei sich erste spektrale Informationen des ersten Beobachtungsbildes zur Anzeige und zweite spektrale Informationen des zweiten Beobachtungsbildes zur Anzeige voneinander unterscheiden;
eine Lichtquelleneinheit (20), die so konfiguriert ist, dass sie erstes Beleuchtungslicht, das die ersten spektralen Informationen aufweist, und zweites Beleuchtungslicht, das die zweiten spektralen Informationen aufweist, emittiert;
eine Lichtquellensteuereinheit (21), die so konfiguriert ist, dass sie veranlasst, dass jedes des ersten Beleuchtungslichts und des zweiten Beleuchtungslichts für einen Lichtemissionszeitraum emittiert wird, und das erste Beleuchtungslicht und das zweite Beleuchtungslicht automatisch umschaltet,
wobei das erste Beobachtungsbild zur Anzeige in einem Fall erhalten wird, in dem ein Bild des zu beobachtenden Objekts, das mit dem ersten Beleuchtungslicht beleuchtet wird, aufgenommen wird;
das zweite Beobachtungsbild zur Anzeige in einem Fall erhalten wird, in dem ein Bild des zu beobachtenden Objekts, das mit dem zweiten Beleuchtungslicht beleuchtet wird, aufgenommen wird;
**dadurch gekennzeichnet, dass**
der Lichtemissionszeitraum mindestens zwei oder mehr Einzelbilder beträgt,
wobei die Lichtquellensteuereinheit (21) so konfiguriert ist, dass sie den Farbton mindestens eines Teils einer Hintergrundschleimhaut des ersten Beobachtungsbildes zur Anzeige mit dem Farbton mindestens eines Teils einer Hintergrundschleimhaut des zweiten Beobachtungsbildes zur Anzeige gleichmacht, indem sie Lichtintensitätsverhältnisse von grünem Licht und rotem Licht im ersten Beleuchtungslicht mit Lichtintensitätsverhältnissen von grünem Licht und rotem Licht im zweiten Beleuchtungslicht gleichmacht.

2. Endoskopsystem nach Anspruch 1,
wobei die Anzeigesteuereinheit (66) so konfiguriert ist, dass sie die Anzeigeeinheit veranlasst, ein drittes Beobachtungsbild zur Anzeige, das sich vom ersten Beobachtungsbild zur Anzeige und dem zweiten Beobachtungsbild zur Anzeige unterscheidet, zu einem Zeitpunkt anzuzeigen, wenn das erste Beobachtungsbild zur Anzeige und das zweite Beobachtungsbild zur Anzeige umgeschaltet werden.

3. Endoskopsystem nach Anspruch 2,
wobei die Lichtquellensteuereinheit (21) so konfiguriert ist, dass sie veranlasst, dass drittes Beleuchtungslicht, das sich vom ersten Beleuchtungslicht und dem zweiten Beleuchtungslicht unterscheidet, zu einem Zeitpunkt emittiert wird, wenn das erste Beobachtungsbild zur Anzeige und das zweite Beobachtungsbild zur Anzeige umgeschaltet werden.

4. Endoskopsystem nach einem der vorstehenden Ansprüche,
wobei das erste Beleuchtungslicht violettes Licht, grünes Licht und rotes Licht beinhaltet, und
das zweite Beleuchtungslicht grünes Licht und rotes Licht beinhaltet.

5. Endoskopsystem nach einem der vorstehenden Ansprüche,
wobei das erste Beleuchtungslicht violettes Licht, grünes Licht und rotes Licht beinhaltet, und
das zweite Beleuchtungslicht blaues Licht, grünes Licht und rotes Licht beinhaltet.

6. Endoskopsystem nach einem der vorstehenden Ansprüche,
wobei das erste Beobachtungsbild zur Anzeige erste spezifische Informationen, die in einer ersten Tiefe von biologischem Gewebe beinhaltet sind, in einem anderen Bereich davon als der Hintergrundschleimhaut beinhaltet,
das zweite Beobachtungsbild zur Anzeige zweite spezifische Informationen, die in einer zweiten Tiefe des biologischen Gewebes beinhaltet sind, in einem anderen Bereich davon als der Hintergrundschleimhaut beinhaltet, und
die zweite Tiefe tiefer ist als die erste Tiefe.

7. Endoskopsystem nach Anspruch 6,
wobei das erste Beleuchtungslicht eine erste Tiefe erreichendes Licht beinhaltet, das die erste Tiefe erreicht, und das zweite Beleuchtungslicht eine zweite Tiefe erreichendes Licht beinhaltet, das die zweite Tiefe erreicht.

8. Endoskopsystem nach Anspruch 1, weiter umfassend:
eine Beobachtungsbild-zur-Anzeige-Verarbeitungseinheit, die so konfiguriert ist, dass sie ein Beobachtungsbild zur Anzeige auf Grundlage des Beobachtungsbildes erzeugt,
wobei die Beobachtungsbild-zur-Anzeige-Verarbeitungseinheit so konfiguriert ist, dass sie ein erstes Farbsignal des Beobachtungsbildes Helligkeitsinformationen zuordnet, um das erste Beobachtungsbild zur Anzeige als Beobachtungsbild zur Anzeige zu erzeugen, und ein zweites Farbsignal des Beobachtungsbildes Helligkeitsinformationen zuordnet, um das zweite Beobachtungsbild zur Anzeige als Beobachtungsbild zur Anzeige zu erzeugen.

9. Endoskopsystem nach Anspruch 8,
wobei das Beobachtungsbild in einem Fall erhalten wird, in dem ein Bild des zu beobachtenden Objekts, das mit speziellem Licht beleuchtet wird, welches violettes Licht, grünes Licht und rotes Licht beinhaltet, aufgenommen wird.

10. Endoskopsystem nach Anspruch 8 oder 9,
wobei die Anzeigesteuereinheit (66) so konfiguriert ist, dass sie die Anzeigeeinheit veranlasst, ein drittes Beobachtungsbild zur Anzeige, das sich vom ersten Beobachtungsbild zur Anzeige und dem zweiten Beobachtungsbild zur Anzeige unterscheidet, zu einem Zeitpunkt anzuzeigen, wenn das erste Beobachtungsbild zur Anzeige und das zweite Beobachtungsbild zur Anzeige umgeschaltet werden.

11. Endoskopsystem nach Anspruch 10,
wobei die Beobachtungsbild-zur-Anzeige-Verarbeitungseinheit so konfiguriert ist, dass sie ein zusammengesetztes Signal, das durch Kombination des ersten Farbsignals und des zweiten Farbsignals des Beobachtungsbildes erhalten wird, Helligkeitsinformationen zuordnet, um das dritte Beobachtungsbild zur Anzeige zu einem Zeitpunkt zu erzeugen, wenn das erste Beobachtungsbild zur Anzeige und das zweite Beobachtungsbild zur Anzeige umgeschaltet werden.

12. Endoskopsystem nach einem der Ansprüche 8 bis 11,
wobei das erste Beobachtungsbild zur Anzeige erste spezifische Informationen, die in einer ersten Tiefe von biologischem Gewebe beinhaltet sind, in einem anderen Bereich davon als der Hintergrundschleimhaut beinhaltet,
das zweite Beobachtungsbild zur Anzeige zweite spezifische Informationen, die in einer zweiten Tiefe des biologischen Gewebes beinhaltet sind, in einem anderen Bereich davon als der Hintergrundschleimhaut beinhaltet, und
die zweite Tiefe tiefer ist als die erste Tiefe.

13. Endoskopsystem nach Anspruch 12,
wobei die ersten spektralen Informationen spektrale Informationen einer ersten Tiefe beinhalten, die der ersten Tiefe entsprechen, und die zweiten spektralen Informationen spektrale Informationen einer zweiten Tiefe beinhalten, die der zweiten Tiefe entsprechen.

14. Endoskopsystem nach einem der Ansprüche 1 bis 13,
wobei ein Parameter für das erste Beobachtungsbild zur Anzeige, der für die Erzeugung des ersten Beobachtungsbildes zur Anzeige verwendet wird, und ein Parameter für das zweite Beobachtungsbild zur Anzeige, der für die Erzeugung des zweiten Beobachtungsbildes zur Anzeige verwendet wird, gemäß dem Umschalten des ersten Beobachtungsbildes zur Anzeige und des zweiten Beobachtungsbildes zur Anzeige umgeschaltet werden.

15. Endoskopsystem nach einem der Ansprüche 1 bis 5, 8 bis 11 und 14,
wobei das erste Beobachtungsbild zur Anzeige erste spezifische Informationen, die in einer ersten Tiefe von biologischem Gewebe beinhaltet sind, in einem anderen Bereich davon als der Hintergrundschleimhaut beinhaltet, und das zweite Beobachtungsbild zur Anzeige zweite spezifische Informationen, die in einer zweiten Tiefe des biologischen Gewebes beinhaltet sind, welche tiefer ist als die erste Tiefe, in einem anderen Bereich davon als der Hintergrundschleimhaut beinhaltet, und
erste Reduktionsverarbeitung zum Reduzieren der zweiten spezifischen Informationen aus dem ersten Beobachtungsbild zur Anzeige oder zweite Reduktionsverarbeitung zum Reduzieren der ersten spezifischen Informationen aus dem zweiten Beobachtungsbild zur Anzeige durchgeführt wird.

16. Endoskopsystem nach einem der Ansprüche 1 bis 15,
wobei die Anzeigesteuereinheit (66) eine Anzeigezeitraum-Einstelleinheit (66a) beinhaltet, die einen Anzeigezeitraum des ersten Beobachtungsbildes zur Anzeige und einen Anzeigezeitraum des zweiten Beobachtungsbildes zur Anzeige einstellt.

17. Endoskopsystem nach einem der Ansprüche 1 bis 16, weiter umfassend:
eine Standbild-Erfassungsanweisungseinheit (13b), die so konfiguriert ist, dass sie eine Standbilderfassungsanweisung ausgibt; und
eine Standbild-Speichersteuereinheit (68), die so konfiguriert ist, dass sie in einem Fall, in dem die Standbilderfassungsanweisung ausgegeben wird, Steuerung durchführt, um einen Satz von Beobachtungsbildern zur Anzeige, der das erste Beobachtungsbild zur Anzeige und das zweite Beobachtungsbild zur Anzeige beinhaltet, in einer Standbildspeichereinheit (67) zu speichern.

18. Endoskopsystem nach einem der Ansprüche 6, 7, 12, 13 und 15,
wobei die ersten spezifischen Informationen Blutgefäße in einer ersten Tiefe sind, die in der ersten Tiefe positioniert sind, und die zweiten spezifischen Informationen Blutgefäße in einer zweiten Tiefe sind, die in der zweiten Tiefe positioniert sind.

19. Endoskopsystem nach Anspruch 18,
wobei die Blutgefäße in der ersten Tiefe, die durch Blutgefäß-Extraktionsverarbeitung extrahiert werden, im ersten Beobachtungsbild zur Anzeige angezeigt werden, und die Blutgefäße in der zweiten Tiefe, die durch die Blutgefäß-Extraktionsverarbeitung extrahiert werden, im zweiten Beobachtungsbild zur Anzeige angezeigt werden.

20. Endoskopsystem nach einem der Ansprüche 6, 7, 12, 13 und 15,
wobei die ersten spezifischen Informationen Drüsenstrukturen in einer ersten Tiefe sind, die in der ersten Tiefe positioniert sind, und die zweiten spezifischen Informationen Drüsenstrukturen in einer zweiten Tiefe sind, die in der zweiten Tiefe positioniert sind.

## Revendications

1. Système d'endoscope comprenant :
une unité d'acquisition d'image (53) configurée pour acquérir une image d'observation ;
une unité de commande d'affichage (66) configurée pour amener une unité d'affichage à commuter automatiquement et afficher au moins deux première et deuxième images d'observation acquises par l'unité d'acquisition d'image (66) à afficher, dans lequel le même objet à observer est affiché, pendant une durée d'affichage d'au moins deux trames ou plus,
dans lequel les premières informations spectrales de la première image d'observation à afficher et les secondes informations spectrales pour la deuxième image d'observation à afficher sont différentes les unes des autres ;
une unité de source de lumière (20) configurée pour émettre une première source de lumière présentant les premières informations spectrales et une seconde source de lumière présentant les secondes informations spectrales ;
une unité de commande de source de lumière (21) configurée pour amener chacune de la première source de lumière et de la seconde source de lumière à être émise pendant une période d'émission de lumière et qui commute automatiquement la première source de lumière avec la seconde source de lumière,
dans lequel la première image d'observation à afficher est obtenue dans un cas où une image de l'objet à observer éclairée avec la première source de lumière est détectée ;
dans lequel la deuxième image d'observation à afficher est obtenue dans un cas où une image de l'objet à observer éclairée avec la seconde source de lumière est détectée ;
**caractérisé en ce que**
la période d'émission de lumière est d'au moins deux trames ou plus,
dans lequel l'unité de commande de source de lumière (21) est configurée pour faire que la teinte d'au moins une partie d'une membrane muqueuse de fond de la première image d'observation à afficher soit identique à la teinte d'au moins une partie d'une membrane muqueuse de fond de la deuxième image d'observation à afficher en faisant en sorte que les rapports d'intensité lumineuse de la lumière verte et la lumière rouge dans la première source de lumière soient identiques aux rapports d'intensité lumineuse de la lumière verte et la lumière rouge dans la seconde source de lumière.

2. Système d'endoscope selon la revendication 1,
dans lequel l'unité de commande d'affichage (66) est configurée pour que l'unité d'affichage affiche une troisième image d'observation à afficher différente de la première image d'observation à afficher et de la deuxième image d'observation à afficher à un moment où la première image d'observation à afficher et la deuxième image d'observation à afficher sont commutées.

3. Système d'endoscope selon la revendication 2,
dans lequel l'unité de commande de source de lumière (21) est configurée pour que la troisième source de lumière différente de la première source de lumière et de la deuxième source de lumière soit émise lorsque la première image d'observation à afficher et la deuxième image d'observation à afficher sont commutées.

4. Système d'endoscope selon l'une quelconque des revendications précédentes,
dans lequel la première source de lumière inclut une lumière violette, une lumière verte, et une lumière rouge, et
la seconde source de lumière inclut une lumière verte et une lumière rouge.

5. Système d'endoscope selon l'une quelconque des revendications précédentes,
dans lequel la première source de lumière inclut une lumière violette, une lumière verte, et une lumière rouge, et
la seconde source de lumière inclut une lumière bleue, une lumière verte et une lumière rouge.

6. Système d'endoscope selon l'une quelconque des revendications précédentes,
dans lequel la première image d'observation à afficher inclut des premières informations spécifiques, qui sont incluses dans une première profondeur du tissu biologique, dans une région de celui-ci autre que la membrane muqueuse de fond,
la deuxième image d'observation à afficher inclut des secondes informations spécifiques, qui sont incluses dans une seconde profondeur du tissu biologique, dans une région de celui-ci autre que la membrane muqueuse de fond,
la seconde profondeur est plus profonde que la première profondeur.

7. Système d'endoscope selon la revendication 6,
dans lequel la première source de lumière inclut une première lumière atteignant les profondeurs atteignant la première profondeur, et la seconde source de lumière inclut une seconde lumière atteignant les profondeurs atteignant la seconde profondeur.

8. Système d'endoscope selon la revendication 1, comprenant en outre :
une unité de traitement d'image d'observation à afficher configurée pour générer une image d'observation à afficher sur la base de l'image d'observation,
dans lequel l'unité de traitement d'image d'observation à afficher est configurée pour attribuer un premier signal de couleur de l'image d'observation aux informations de luminosité pour générer la première image d'observation à afficher en tant que l'image d'observation à afficher, et pour attribuer un second signal de couleur de l'image d'observation aux informations de luminosité pour générer la deuxième image d'observation à afficher en tant que l'image d'observation à afficher.

9. Système d'endoscope selon la revendication 8,
dans lequel l'image d'observation est obtenue dans un cas où une image de l'objet à observer éclairée avec une lumière spécifique incluant une lumière violette, une lumière verte, et une lumière rouge est détectée.

10. Système d'endoscope selon la revendication 8 ou 9,
dans lequel l'unité de commande d'affichage (66) est configurée pour que l'unité d'affichage affiche une troisième image d'observation à afficher différente de la première image d'observation à afficher et de la deuxième image d'observation à afficher à un moment où la première image d'observation à afficher et la deuxième image d'observation à afficher sont commutées.

11. Système d'endoscope selon la revendication 10,
dans lequel l'unité de traitement d'image d'observation à afficher est configurée pour attribuer un signal composite, qui est obtenu par la combinaison du premier signal de couleur et du second signal de couleur de l'image d'observation, aux informations de luminosité pour générer la troisième image d'observation à afficher lorsque la première image d'observation à afficher et la deuxième image d'observation à afficher sont commutées.

12. Système d'endoscope selon l'une quelconque des revendications 8 à 11,
dans lequel la première image d'observation à afficher inclut des premières informations spécifiques, qui sont incluses dans une première profondeur du tissu biologique, dans une région de celui-ci autre que la membrane muqueuse de fond,
la deuxième image d'observation à afficher inclut des secondes informations spécifiques, qui sont incluses dans une seconde profondeur du tissu biologique, dans une région de celui-ci autre que la membrane muqueuse de fond,
la seconde profondeur est plus profonde que la première profondeur.

13. Système d'endoscope selon la revendication 12,
dans lequel les premières informations spectrales incluent des premières informations spectrales de profondeur correspondant à la première profondeur et les secondes informations spectrales incluent des informations spectrales de profondeur correspondant à la seconde profondeur.

14. Système d'endoscope selon l'une quelconque des revendications 1 à 13,
dans lequel un paramètre pour la première image d'observation à afficher utilisé pour la génération de la première image d'observation à afficher et un paramètre de la deuxième image d'observation à afficher utilisé pour la génération de la deuxième image d'observation à afficher sont commutés conformément à la commutation de la première image d'observation à afficher et la deuxième image d'observation à afficher.

15. Système d'endoscope selon l'une quelconque des revendications 1 à 5, 8 à 11 et 14,
dans lequel la première image d'observation à afficher inclut des premières informations spécifiques, qui sont incluses dans une première profondeur du tissu biologique, dans une région de celui-ci autre que la membrane muqueuse de fond et la deuxième image d'observation à afficher inclut des secondes informations spécifiques, qui sont incluses dans une seconde profondeur du tissu biologique plus profonde que la première profondeur, dans une région de celui-ci autre que la membrane muqueuse de fond, et
un premier traitement de réduction pour réduire les secondes informations spécifiques de la première image d'observation à afficher ou un second traitement de réduction pour réduire les premières informations spécifiques de la deuxième image d'observation à afficher est effectué.

16. Système d'endoscope selon l'une quelconque des revendications 1 à 15,
dans lequel l'unité de commande d'affichage (66) inclut une unité d'affichage définie sur une période (66a) qui définit une période d'affichage de la première image d'observation à afficher et une période d'affichage de la deuxième image d'observation à afficher.

17. Système d'endoscope selon l'une quelconque des revendications 1 à 16, comprenant en outre :
une unité d'instruction d'acquisition d'image statique (13b) est configurée pour délivrer une instruction d'acquisition d'image statique ; et
une unité de commande de stockage d'image statique (68) est configurée pour effectuer une commande pour stocker un ensemble d'images d'observation à afficher, qui inclut la première image d'observation à afficher et la deuxième image d'observation à afficher, dans une unité de stockage d'image statique (67) dans un cas où l'instruction d'acquisition d'image statique est délivrée.

18. Système d'endoscope selon l'une quelconque des revendications 6, 7, 12, 13, et 15,
dans lequel les premières informations spécifiques sont des vaisseaux sanguins en première profondeur positionnés à une première profondeur, et les secondes informations spécifiques sont des vaisseaux sanguins en seconde profondeur positionnés à une seconde profondeur.

19. Système d'endoscope selon la revendication 18,
dans lequel les vaisseaux sanguins en première profondeur extraits par un processus d'extraction de vaisseaux sanguins sont affichés dans la première image d'observation à afficher, et les vaisseaux sanguins en seconde profondeur extraits par un processus d'extraction de vaisseaux sanguins sont affichés dans la deuxième image d'observation à afficher.

20. Système d'endoscope selon l'une quelconque des revendications 6, 7, 12, 13, et 15,
dans lequel les premières informations spécifiques sont des structures glandulaires en première profondeur positionnées à une première profondeur, et les secondes informations spécifiques sont des structures glandulaires en seconde profondeur positionnées à une seconde profondeur.
